# EUROPEAN PATENT APPLICATION

(11) **EP 4 644 422 A1**
(43) Date of publication of application: **05.11.2025**
(21) Application number: 23910738.6
(22) Date of filing: 27.12.2023
(51) Int. Cl.: C07K 16/28, A61P 35/00

(54) **ANTI-NKG2A ANTIBODY AND USE THEREOF**

(30) Priority: 28.12.2022 CN 202211699426
(71) Applicant: BioRay Pharmaceutical Co., Ltd., Jiaojiang District, Taizhou Zhejiang 318000 (CN); Bioray Pharmaceutical (Hangzhou) Co., Ltd., Hangzhou, Zhejiang 311400 (CN)
(72) Inventor: ZHOU, Yaqiong, Hangzhou, Zhejiang 311400 (CN); NIE, Lei, Taizhou, Zhejiang 318000 (CN); XU, Tong, Hangzhou, Zhejiang 311400 (CN); WU, Zhenhua, Taizhou, Zhejiang 318000 (CN); LI, Na, Taizhou, Zhejiang 318000 (CN); WANG, Haibin, Hangzhou, Zhejiang 311400 (CN); GAO, Zhangzhao, Hangzhou, Zhejiang 311400 (CN); QI, Jian, Hangzhou, Zhejiang 311400 (CN); ZHU, Shanshan, Hangzhou, Zhejiang 311400 (CN)
(74) Representative: Dehns
(86) International application number: PCT/CN2023/142410
(87) International publication number: WO 2024/140820

(57) **Abstract**

Provided are an anti-NKG2A antibody or an antigen-binding fragment thereof and use thereof, wherein the antibody or the antigen-binding fragment thereof comprises VH-CDR1, VH-CDR2, VH-CDR3, VL-CDR1, VL-CDR2 and VL-CDR3. The antibody or the antigen-binding fragment thereof has the potential to treat tumors and provides an option for clinical medication in tumor immunotherapy.

## Description

### TECHNICAL FIELD

The present disclosure belongs to the field of biomedicine and relates to an anti-NKG2A antibody.

### BACKGROUND

NKG2A, also known as natural killer cell lectin-Like receptor, is an inhibitory receptor in the NKG2 receptor family, mainly expressed on the surface of NK cells, CD8⁺ T cells, Th2 cells and NKT cells. On the surface of human immune cells, molecules of NKG2A and CD94 (a membrane protein on the surface of NK cells) are linked by disulfide bonds to form a heterodimeric complex NKG2A-CD94, which is recognized by the non-classical major histocompatibility complex class I ( MHC I) molecule HLA-E on the target cells. HLA-E is the only ligand of the heterodimeric receptor CD94/NKG2A and is a non-classical MHC class Ib molecule. The expression of such molecule is low under normal conditions, but a variety of tumor cells highly express HLA-E, and NKG2A is highly expressed by NK and CD8⁺ T cells at the tumor infiltration site, allowing the tumor cells to escape immune recognition by NK and CD8⁺ T cells. In addition, after viral infection, host cells can be induced to express ligands that bind to the inhibitory receptor NKG2A of NK and CD8⁺ T cells, thereby escaping clearance by the immune system (Zheng M et al., Cellular and Molecular Immunology, 2020, 17(5)). Therefore, anti-NKG2A antibodies can release the killing activity of NK by blocking this inhibitory signal,thereby playing an anti-tumor role.

### SUMMARY

The present disclosure provides an antibody that is capable of specifically binding to NKG2A on the surface of human NK92 cells, and is capable of blocking the NKG2A and HLA-E signaling pathways, significantly increasing the killing activity of NK92 cells or primary NK cells on HLA-E positive tumor cells. The antibody has good anti-tumor activity, and has better functional activity than known anti-NKG2A antibodies. Therefore, the antibody of the present disclosure has the potential to treat tumors and provides an option for clinical medication in tumor immunotherapy.

In a first aspect, the present disclosure provides an anti-NGK2A antibody or an antigen-binding fragment thereof, comprising a heavy chain variable region and a light chain variable region, the heavy chain variable region comprises three CDRs: VH-CDR1, VH-CDR2 and VH-CDR3, and the light chain variable region comprises three CDRs: VL-CDR1, VL-CDR2 and VL-CDR3; wherein,
the VH-CDR1 has an amino acid sequence as set forth in SEQ ID NO: 2;
the VH-CDR2 has an amino acid sequence as set forth in SEQ ID NO: 3;
the VH-CDR3 has an amino acid sequence as set forth in SEQ ID NO: 4;
the VL-CDR1 has an amino acid sequence as set forth in SEQ ID NO: 6;
the VL-CDR2 has an amino acid sequence as set forth in SEQ ID NO: 7;
the VL-CDR3 has an amino acid sequence as set forth in SEQ ID NO: 8; or
the VH-CDR1 has an amino acid sequence as set forth in SEQ ID NO: 10;
the VH-CDR2 has an amino acid sequence as set forth in SEQ ID NO: 11;
the VH-CDR3 has an amino acid sequence as set forth in SEQ ID NO: 12;
the VL-CDR1 has an amino acid sequence as set forth in SEQ ID NO: 14;
the VL-CDR2 has an amino acid sequence as set forth in SEQ ID NO: 15;
the VL-CDR3 has an amino acid sequence as set forth in SEQ ID NO: 16; or
the VH-CDR1 has an amino acid sequence as set forth in SEQ ID NO: 18;
the VH-CDR2 has an amino acid sequence as set forth in SEQ ID NO: 19;
the VH-CDR3 has an amino acid sequence as set forth in SEQ ID NO: 20;
the VL-CDR1 has an amino acid sequence as set forth in SEQ ID NO: 22;
the VL-CDR2 has an amino acid sequence as set forth in SEQ ID NO: 23;
the VL-CDR3 has an amino acid sequence as set forth in SEQ ID NO: 24; or
the VH-CDR1 has an amino acid sequence as set forth in SEQ ID NO: 26;
the VH-CDR2 has an amino acid sequence as set forth in SEQ ID NO: 27;
the VH-CDR3 has an amino acid sequence as set forth in SEQ ID NO: 28;
the VL-CDR1 has an amino acid sequence as set forth in SEQ ID NO: 30;
the VL-CDR2 has an amino acid sequence as set forth in SEQ ID NO: 31;
the VL-CDR3 has an amino acid sequence as set forth in SEQ ID NO: 32; or
the VH-CDR1 has an amino acid sequence as set forth in SEQ ID NO: 34;
the VH-CDR2 has an amino acid sequence as set forth in SEQ ID NO: 35;
the VH-CDR3 has an amino acid sequence as set forth in SEQ ID NO: 36;
the VL-CDR1 has an amino acid sequence as set forth in SEQ ID NO: 38;
the VL-CDR2 has an amino acid sequence as set forth in SEQ ID NO: 39;
the VL-CDR3 has an amino acid sequence as set forth in SEQ ID NO: 40; or
the VH-CDR1 has an amino acid sequence as set forth in SEQ ID NO: 42;
the VH-CDR2 has an amino acid sequence as set forth in SEQ ID NO: 43;
the VH-CDR3 has an amino acid sequence as set forth in SEQ ID NO: 44;
the VL-CDR1 has an amino acid sequence as set forth in SEQ ID NO: 46;
the VL-CDR2 has an amino acid sequence as set forth in SEQ ID NO: 47;
the VL-CDR3 has an amino acid sequence as set forth in SEQ ID NO: 48; or
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 54;
the VL-CDR2 has an amino acid sequence as set forth in SEQ ID NO: 55;
the VL-CDR3 has an amino acid sequence as set forth in SEQ ID NO: 56; or
the VH-CDR1 has an amino acid sequence as set forth in SEQ ID NO: 58;
the VH-CDR2 has an amino acid sequence as set forth in SEQ ID NO: 59;
the VH-CDR3 has an amino acid sequence as set forth in SEQ ID NO: 60;
the VL-CDR1 has an amino acid sequence as set forth in SEQ ID NO: 62;
the VL-CDR2 has an amino acid sequence as set forth in SEQ ID NO: 63;
the VL-CDR3 has an amino acid sequence as set forth in SEQ ID NO: 64; or
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 68;
the VL-CDR2 has an amino acid sequence as set forth in SEQ ID NO: 55;
the VL-CDR3 has an amino acid sequence as set forth in SEQ ID NO: 56;
when the VL-CDR1 has an amino acid sequence as set forth in SEQ ID NO: 68, X₁ is T, S, Q, A, D, G, E, F, I, K, L, P, V, W, Y, R or H; X₂ is N, Q, S or T; X₃ is G, A, L, E, K, R, S, N, T or Q; wherein, one and only one of the two conditions must exist: X₂ is N or X₃ is G .

In some embodiments, the antibody or the antigen-binding fragment thereof comprises a heavy chain variable region and a light chain variable region, the heavy chain variable region comprises three CDRs: VH-CDR1, VH-CDR2 and VH-CDR3, and the light chain variable region comprises three CDRs: VL-CDR1, VL-CDR2 and VL-CDR3; wherein,
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 100, 102, 104, 106, 108, 110, 112, 114, 116, 118, 120, 122, 124, 126, 128, 130, 132, 134, 136, 138, 140, 142, 144, 146, 148, 150, 152, 154, 156, 158 or 160;
the VL-CDR2 has an amino acid sequence as set forth in SEQ ID NO: 55; and
the VL-CDR3 has an amino acid sequence as set forth in SEQ ID NO: 56.

In some embodiments, the antibody or the antigen-binding fragment thereof comprises a heavy chain variable region and a light chain variable region, the heavy chain variable region comprises three CDRs: VH-CDR1, VH-CDR2 and VH-CDR3, and the light chain variable region comprises three CDRs: VL-CDR1, VL-CDR2 and VL-CDR3; wherein,
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 104;
the VL-CDR2 has an amino acid sequence as set forth in SEQ ID NO: 55; and
the VL-CDR3 has an amino acid sequence as set forth in SEQ ID NO: 56.

In some embodiments, the antibody or the antigen-binding fragment thereof comprises a heavy chain variable region and a light chain variable region, the heavy chain variable region comprises three CDRs: VH-CDR1, VH-CDR2 and VH-CDR3, and the light chain variable region comprises three CDRs: VL-CDR1, VL-CDR2 and VL-CDR3; wherein,
the VH-CDR1 has an amino acid sequence as set forth in SEQ ID NO: 58;
the VH-CDR2 has an amino acid sequence as set forth in SEQ ID NO: 59;
the VH-CDR3 has an amino acid sequence as set forth in SEQ ID NO: 60;
the VL-CDR1 has an amino acid sequence as set forth in SEQ ID NO: 62;
the VL-CDR2 has an amino acid sequence as set forth in SEQ ID NO: 63;
the VL-CDR3 has an amino acid sequence as set forth in SEQ ID NO: 64.

In some embodiments, the present disclosure provides an anti-NGK2A antibody or an antigen-binding fragment thereof comprising a heavy chain variable region and a light chain variable region, wherein:
the heavy chain variable region comprises three complementarity determining regions (CDRs) of a heavy chain variable region with an amino acid sequence as set forth in SEQ ID NO: 1, and the light chain variable region comprises three complementarity determining regions (CDRs) of a light chain variable region with an amino acid sequence as set forth in SEQ ID NO: 5; or
the heavy chain variable region comprises three complementarity determining regions (CDRs) of a heavy chain variable region with an amino acid sequence as set forth in SEQ ID NO: 9, and the light chain variable region comprises three complementarity determining regions (CDRs) of a light chain variable region with an amino acid sequence as set forth in SEQ ID NO: 13; or
the heavy chain variable region comprises three complementarity determining regions (CDRs) of a heavy chain variable region with an amino acid sequence as set forth in SEQ ID NO: 17, and the light chain variable region comprises three complementarity determining regions (CDRs) of a light chain variable region with an amino acid sequence as set forth in SEQ ID NO: 21; or
the heavy chain variable region comprises three complementarity determining regions (CDRs) of a heavy chain variable region with an amino acid sequence as set forth in SEQ ID NO: 25, and the light chain variable region comprises three complementarity determining regions (CDRs) of a light chain variable region with an amino acid sequence as set forth in SEQ ID NO: 29; or
the heavy chain variable region comprises three complementarity determining regions (CDRs) of a heavy chain variable region with an amino acid sequence as set forth in SEQ ID NO: 33, and the light chain variable region comprises three complementarity determining regions (CDRs) of a light chain variable region with an amino acid sequence as set forth in SEQ ID NO: 37; or
the heavy chain variable region comprises three complementarity determining regions (CDRs) of a heavy chain variable region with an amino acid sequence as set forth in SEQ ID NO: 41, and the light chain variable region comprises three complementarity determining regions (CDRs) of a light chain variable region with an amino acid sequence as set forth in SEQ ID NO: 45; or
the heavy chain variable region comprises three complementarity determining regions (CDRs) of a heavy chain variable region with an amino acid sequence as set forth in SEQ ID NO: 49, and the light chain variable region comprises three complementarity determining regions (CDRs) of a light chain variable region with an amino acid sequence as set forth in SEQ ID NO: 53; or
the heavy chain variable region comprises three complementarity determining regions (CDRs) of a heavy chain variable region with an amino acid sequence as set forth in SEQ ID NO: 57, and the light chain variable region comprises three complementarity determining regions (CDRs) of a light chain variable region with an amino acid sequence as set forth in SEQ ID NO: 61; or
the heavy chain variable region comprises three complementarity determining regions (CDRs) of a heavy chain variable region with an amino acid sequence as set forth in SEQ ID NO: 65, 85 or 86, and the light chain variable region comprises three complementarity determining regions (CDRs) of a light chain variable region with an amino acid sequence as set forth in SEQ ID NO: 66, 87, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159; or
the heavy chain variable region comprises three complementarity determining regions (CDRs) of a heavy chain variable region with an amino acid sequence as set forth in SEQ ID NO: 89, 90, or 91, and the light chain variable region comprises three complementarity determining regions (CDRs) of a light chain variable region with an amino acid sequence as set forth in SEQ ID NO: 92, 93, or 94.

In some embodiments, the antibody or the antigen-binding fragment thereof comprises a heavy chain variable region and a light chain variable region, wherein the heavy chain variable region comprises three complementarity determining regions (CDRs) of a heavy chain variable region with an amino acid sequence as set forth in SEQ ID NO: 65, and the light chain variable region comprises three complementarity determining regions (CDRs) of a light chain variable region with an amino acid sequence as set forth in SEQ ID NO: 103.

In some embodiments, the antibody or the antigen-binding fragment thereof comprises a heavy chain variable region and a light chain variable region, wherein the heavy chain variable region comprises three complementarity determining regions (CDRs) of a heavy chain variable region having an amino acid sequence as set forth in SEQ ID NO: 91, and the light chain variable region comprises three complementarity determining regions (CDRs) of a light chain variable region having an amino acid sequence as set forth in SEQ ID NO: 92.

Those skilled in the art will easily identify the CDRs defined by each numbering system. Moreover, the correspondence between different numbering systems is well known to those skilled in the art. In some embodiments, the CDRs are defined according to the Kabat, IMGT, Chothia, AbM or Contact numbering conventions. In some embodiments, the CDRs are defined according to the Kabat, IMGT or Chothia numbering conventions.

In preferred embodiments, the heavy chain variable region of the antibody or the antigen-binding fragment thereof has a sequence as set forth in SEQ ID NO: 1, or a sequence having at least 80%, 85%, 90%, 91%, 92%, 93%, 94%, 95%, 96%, 97%, 98% or 99% sequence identity to the sequence as set forth in SEQ ID NO: 1, and the light chain variable region has a sequence as set forth in SEQ ID NO: 5, or a sequence having at least 80%, 85%, 90%, 91%, 92%, 93%, 94%, 95%, 96%, 97%, 98% or 99% sequence identity to the sequence as set forth in SEQ ID NO: 5; or,
the heavy chain variable region of the antibody or the antigen-binding fragment thereof has a sequence as set forth in SEQ ID NO: 9, or a sequence having at least 80%, 85%, 90%, 91%, 92%, 93%, 94%, 95%, 96%, 97%, 98% or 99% sequence identity to the sequence as set forth in SEQ ID NO: 9, and the light chain variable region has a sequence as set forth in SEQ ID NO: 13, or a sequence having at least 80%, 85%, 90%, 91%, 92%, 93%, 94%, 95%, 96%, 97%, 98% or 99% sequence identity to the sequence as set forth in SEQ ID NO: 13; or,
the heavy chain variable region of the antibody or the antigen-binding fragment thereof has a sequence as set forth in SEQ ID NO: 17, or a sequence having at least 80%, 85%, 90%, 91%, 92%, 93%, 94%, 95%, 96%, 97%, 98% or 99% sequence identity to the sequence as set forth in SEQ ID NO: 17, and the light chain variable region has a sequence as set forth in SEQ ID NO: 21, or a sequence having at least 80%, 85%, 90%, 91%, 92%, 93%, 94%, 95%, 96%, 97%, 98% or 99% sequence identity to the sequence as set forth in SEQ ID NO: 21; or,
the heavy chain variable region of the antibody or the antigen-binding fragment thereof has a sequence as set forth in SEQ ID NO: 25, or a sequence having at least 80%, 85%, 90%, 91%, 92%, 93%, 94%, 95%, 96%, 97%, 98% or 99% sequence identity to the sequence as set forth in SEQ ID NO: 25, and the light chain variable region has a sequence as set forth in SEQ ID NO: 29, or a sequence having at least 80%, 85%, 90%, 91%, 92%, 93%, 94%, 95%, 96%, 97%, 98% or 99% sequence identity to the sequence as set forth in SEQ ID NO: 29; or,
the heavy chain variable region of the antibody or the antigen-binding fragment thereof has a sequence as set forth in SEQ ID NO: 33, or a sequence having at least 80%, 85%, 90%, 91%, 92%, 93%, 94%, 95%, 96%, 97%, 98% or 99% sequence identity to the sequence as set forth in SEQ ID NO: 33, and the light chain variable region has a sequence as set forth in SEQ ID NO: 37, or a sequence having at least 80%, 85%, 90%, 91%, 92%, 93%, 94%, 95%, 96%, 97%, 98% or 99% sequence identity to the sequence as set forth in SEQ ID NO: 37; or,
the heavy chain variable region of the antibody or the antigen-binding fragment thereof has a sequence as set forth in SEQ ID NO: 41, or a sequence having at least 80%, 85%, 90%, 91%, 92%, 93%, 94%, 95%, 96%, 97%, 98% or 99% sequence identity to the sequence as set forth in SEQ ID NO: 41, and the light chain variable region has a sequence as set forth in SEQ ID NO: 45, or a sequence having at least 80%, 85%, 90%, 91%, 92%, 93%, 94%, 95%, 96%, 97%, 98% or 99% sequence identity to the sequence as set forth in SEQ ID NO: 45; or,
the heavy chain variable region of the antibody or the antigen-binding fragment thereof has a sequence as set forth in SEQ ID NO: 49, or a sequence having at least 80%, 85%, 90%, 91%, 92%, 93%, 94%, 95%, 96%, 97%, 98% or 99% sequence identity to the sequence as set forth in SEQ ID NO: 49, and the light chain variable region has a sequence as set forth in SEQ ID NO: 53, or a sequence having at least 80%, 85%, 90%, 91%, 92%, 93%, 94%, 95%, 96%, 97%, 98% or 99% sequence identity to the sequence as set forth in SEQ ID NO: 53; or,
the heavy chain variable region of the antibody or the antigen-binding fragment thereof has a sequence as set forth in SEQ ID NO: 57, or a sequence having at least 80%, 85%, 90%, 91%, 92%, 93%, 94%, 95%, 96%, 97%, 98% or 99% sequence identity to the sequence as set forth in SEQ ID NO: 57, and the light chain variable region has a sequence as set forth in SEQ ID NO: 61, or a sequence having at least 80%, 85%, 90%, 91%, 92%, 93%, 94%, 95%, 96%, 97%, 98% or 99% sequence identity to the sequence as set forth in SEQ ID NO: 61; or,
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 66, or a sequence having at least 80%, 85%, 90%, 91%, 92%, 93%, 94%, 95%, 96%, 97%, 98% or 99% sequence identity to the sequence as set forth in SEQ ID NO: 66; or,
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 67, or a sequence having at least 80%, 85%, 90%, 91%, 92%, 93%, 94%, 95%, 96%, 97%, 98% or 99% sequence identity to the sequence as set forth in SEQ ID NO: 67;
when the light chain variable region has an amino acid sequence as set forth in SEQ ID NO: 67, X₁ is T, S, Q, A, D, G, E, F, I, K, L, P, V, W, Y, R or H; X₂ is N, Q, S or T; X₃ is G, A, L, E, K, R, S, N, T or Q; wherein, one and only one of the two conditions must exist: X₂ is N or X₃ is G, or
the heavy chain variable region of the antibody or the antigen-binding fragment thereof has a sequence as set forth in SEQ ID NO: 85, or a sequence having at least 80%, 85%, 90%, 91%, 92%, 93%, 94%, 95%, 96%, 97%, 98% or 99% sequence identity to the sequence as set forth in SEQ ID NO: 85, and the light chain variable region has a sequence as set forth in SEQ ID NO: 87, or a sequence having at least 80%, 85%, 90%, 91%, 92%, 93%, 94%, 95%, 96%, 97%, 98% or 99% sequence identity to the sequence as set forth in SEQ ID NO: 87; or
the heavy chain variable region of the antibody or the antigen-binding fragment thereof has a sequence as set forth in SEQ ID NO: 86, or a sequence having at least 80%, 85%, 90%, 91%, 92%, 93%, 94%, 95%, 96%, 97%, 98% or 99% sequence identity to the sequence as set forth in SEQ ID NO: 86, and the light chain variable region has a sequence as set forth in SEQ ID NO: 87, or a sequence having at least 80%, 85%, 90%, 91%, 92%, 93%, 94%, 95%, 96%, 97%, 98% or 99% sequence identity to the sequence as set forth in SEQ ID NO: 87; or
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 88, or a sequence having at least 80%, 85%, 90%, 91%, 92%, 93%, 94%, 95%, 96%, 97%, 98% or 99% sequence identity to the sequence as set forth in SEQ ID NO: 88; or
the heavy chain variable region of the antibody or the antigen-binding fragment thereof has a sequence as set forth in SEQ ID NO: 89, 90, or 91, or a sequence having at least 80%, 85%, 90%, 91%, 92%, 93%, 94%, 95%, 96%, 97%, 98% or 99% sequence identity to the sequence as set forth in SEQ ID NO: 89, 90, or 91, and the light chain variable region has a sequence as set forth in SEQ ID NO: 92, 93 or 94, or a sequence having at least 80%, 85%, 90%, 91%, 92%, 93%, 94%, 95%, 96%, 97%, 98% or 99% sequence identity to the sequence as set forth in SEQ ID NO: 92, 93 or 94.

In some embodiments, the heavy chain variable region of the antibody or the antigen-binding fragment thereof has a sequence as set forth in SEQ ID NO: 65, and the light chain variable region has a sequence as set forth in SEQ ID NO: 66, 87, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159.

In some embodiments, the heavy chain variable region of the antibody or the antigen-binding fragment thereof has a sequence as set forth in SEQ ID NO: 85, and the light chain variable region has a sequence as set forth in SEQ ID NO: 66, 87, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159.

In some embodiments, the heavy chain variable region of the antibody or the antigen-binding fragment thereof has a sequence as set forth in SEQ ID NO: 86, and the light chain variable region has a sequence as set forth in SEQ ID NO: 66, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159.

In some embodiments, the heavy chain variable region of the antibody or the antigen-binding fragment thereof has a sequence as set forth in SEQ ID NO: 89, 90, or 91, and the light chain variable region has a sequence as set forth in SEQ ID NO: 92, 93, or 94.

In some embodiments, the heavy chain variable region of the antibody or the antigen-binding fragment thereof has a sequence as set forth in SEQ ID NO: 65, and the light chain variable region has a sequence as set forth in SEQ ID NO: 103.

In some embodiments, the heavy chain variable region of the antibody or the antigen-binding fragment thereof has a sequence as set forth in SEQ ID NO: 91, and the light chain variable region has a sequence as set forth in SEQ ID NO: 92.

In some embodiments, the heavy chain variable region of the antibody or the antigen-binding fragment thereof further comprises a human or murine-derived framework region, and/or the light chain variable region of the antibody or the antigen-binding fragment thereof further comprises a human or murine-derived framework region.

In some embodiments, the antibody or the antigen-binding fragment thereof further comprises a heavy chain constant region and/or a light chain constant region; the heavy chain constant region and/or the light chain constant region are human-derived or murine-derived.

In some embodiments, the heavy chain constant region of the antibody is a heavy chain constant region of human IgG4. In some embodiments, the heavy chain constant region of the antibody comprises S228P, L235E, D265A and/or R409K mutations. In some embodiments, the heavy chain constant region comprises the amino acid sequence as set forth in SEQ ID NO: 95 or 161. In some embodiments, the light chain constant region comprises the amino acid sequence as set forth in SEQ ID NO: 96.

In preferred embodiments, the antibody or the antigen-binding fragment thereof is an animal-derived antibody, a chimeric antibody, a humanized antibody or a combination thereof.

In some embodiments, the anti-NGK2A antibody or the antigen-binding fragment thereof, wherein the antibody comprises a heavy chain and a light chain, wherein,
the heavy chain has an amino acid sequence as set forth in SEQ ID NO: 162 or 166, or comprises an amino acid sequence having at least 80%, 85%, 90%, 91%, 92%, 93%, 94%, 95%, 96%, 97%, 98% or 99% sequence identity to SEQ ID NO: 162 or 166, and the light chain has an amino acid sequence as set forth in SEQ ID NO: 163, or comprises an amino acid sequence having at least 80%, 85%, 90%, 91%, 92%, 93%, 94%, 95%, 96%, 97%, 98% or 99% sequence identity to SEQ ID NO: 163.

In some embodiments, the anti-NGK2A antibody or the antigen-binding fragment thereof, wherein the antibody comprises a heavy chain and a light chain, wherein,
the heavy chain has an amino acid sequence as set forth in SEQ ID NO: 164 or 167, or comprises an amino acid sequence having at least 80%, 85%, 90%, 91%, 92%, 93%, 94%, 95%, 96%, 97%, 98% or 99% sequence identity to SEQ ID NO: 164 or 167, and the light chain has an amino acid sequence as set forth in SEQ ID NO: 165, or comprises an amino acid sequence having at least 80%, 85%, 90%, 91%, 92%, 93%, 94%, 95%, 96%, 97%, 98% or 99% sequence identity to SEQ ID NO: 165.

In some embodiments, the anti-NGK2A antibody or the antigen-binding fragment thereof, wherein the antibody comprises a heavy chain and a light chain, wherein, the heavy chain has an amino acid sequence as set forth in SEQ ID NO: 162, and the light chain has an amino acid sequence as set forth in SEQ ID NO: 163.

In some embodiments, the anti-NGK2A antibody or the antigen-binding fragment thereof, wherein the antibody comprises a heavy chain and a light chain, wherein, the heavy chain has an amino acid sequence as set forth in SEQ ID NO: 164, and the light chain has an amino acid sequence as set forth in SEQ ID NO: 165.

In some embodiments, the anti-NGK2A antibody or the antigen-binding fragment thereof, wherein the antibody comprises a heavy chain and a light chain, wherein, the heavy chain has an amino acid sequence as set forth in SEQ ID NO: 166, and the light chain has an amino acid sequence as set forth in SEQ ID NO: 163.

In some embodiments, the anti-NGK2A antibody or the antigen-binding fragment thereof, wherein the antibody comprises a heavy chain and a light chain, wherein, the heavy chain has an amino acid sequence as set forth in SEQ ID NO: 167, and the light chain has an amino acid sequence as set forth in SEQ ID NO: 165.

In some embodiments, the antibody or the antigen-binding fragment thereof is an IgG antibody, a diabody, a single-chain antibody, a bispecific antibody, a single-domain antibody, or a multispecific antibody.

In some embodiments, the antibody or the antigen-binding fragment thereof is a full-length antibody protein, or an antigen-binding fragment.

In some embodiments, the antigen-binding fragment is selected from Fab, Fab', (Fab')₂, Fv, Fvs linked by disulfide bonds, Fd, scFv, and dAb.

In a second aspect, provided is a biological material, which is:
1) a nucleic acid molecule encoding the antibody or the antigen-binding fragment thereof of the present disclosure; preferably, the nucleic acid molecule encodes a heavy chain variable region and/or a light chain variable region of the antibody or the antigen-binding fragment thereof; further preferably, the nucleic acid molecule encodes a heavy chain variable region with a nucleotide sequence as set forth in SEQ ID NO: 69, 71, 73, 75, 77, 79, 81 or 83; and/or, the nucleic acid molecule encodes a light chain variable region with a nucleotide sequence as set forth in SEQ ID NO: 70, 72, 74, 76, 78, 80, 82 or 84;
2) a vector comprising the nucleic acid molecule of 1) above; preferably, the vector is a bacterial plasmid, a phage, a yeast plasmid, a plant cell virus, a mammalian cell virus such as an adenovirus, a retrovirus, or other vectors;
3) a cell comprising the nucleic acid molecule of 1) above or the vector of 2) above; preferably, the cell expresses the antibody or the antigen-binding fragment thereof of the present disclosure. The cell cannot develop into an individual.

In a third aspect, provided is a method for preparing the antibody or the antigen-binding fragment thereof of the present disclosure, which is:
1) chemical synthesis, synthesizing according to the amino acid sequence of the antibody or the antigen-binding fragment thereof; or,
2) biological preparation, including culturing the cell of the second aspect above and harvesting the antibody or the antigen-binding fragment thereof.

In a fourth aspect, provided is a composition, including the anti-NGK2A antibody or the antigen-binding fragment thereof, or the biological material of the present disclosure.

In some embodiments, the composition is a pharmaceutical composition, further including a pharmaceutically acceptable carrier.

In some embodiments, the pharmaceutical composition is a liquid preparation; in some embodiments, the pharmaceutical composition is an injection; in some embodiments, the pharmaceutical composition contains 0.01% ~ 99.99% of the antibody or the antigen-binding fragment thereof of the present disclosure, and the percentage is the mass percentage of the pharmaceutical composition.

In a fifth aspect, provided is a kit, comprising the antibody or the antigen-binding fragment thereof, the biological material, or the antibody composition of the present disclosure; the kit is used for diagnosing or detecting NKG2A protein or cells expressing NKG2A protein in an *ex vivo* sample.

In a sixth aspect, provided is use of the antibody or the antigen-binding fragment thereof, the biological material, or the antibody composition of the present disclosure, wherein the use is:
1) preparing a medicament for preventing and/or treating a disease associated with abnormal expression or abnormal function of NKG2A; or
2) preparing a medicament for enhancing the killing activity of NK92 cells or primary NK cells; or
3) preparing a medicament for stimulating human immune response.

In some embodiments, the disease associated with abnormal expression or abnormal function of NKG2A is selected from a group consisting of cancer, viral disease, autoimmune disease or inflammatory disease; more preferably, the cancer is selected from a group consisting of non-small cell lung cancer, pharyngeal tumor, melanoma, colon cancer, lung cancer, liver cancer, pancreatic cancer, ovarian cancer, endometrial cancer, fallopian tube cancer, bladder cancer, glioma, glioblastoma, thyroid cancer, esophageal cancer, prostate cancer, breast cancer, lymphocytic leukemia, small cell lung cancer, head and neck cancer, head and neck squamous cell carcinoma, kidney cancer, gastric cancer, peritoneal tumor, aleukemic leukemia, acute lymphocytic leukemia, chronic lymphocytic leukemia, B cell lymphoma, T cell lymphoma, Hodgkin lymphoma, non-Hodgkin lymphoma, hairy cell lymphoma, Burketts lymphoma, acute myelogenous leukemia, chronic myelogenous leukemia and promyelocytic leukemia.

In some embodiments, the cancer is a solid tumor, such as non-small cell lung cancer, small cell lung cancer, fallopian tube cancer, peritoneal tumor, ovarian tumor, breast cancer, head and neck squamous cell carcinoma, etc. In some embodiments, the cancer is a cancer expressing HLA-E.

In some embodiments, the cancer is a hematologic tumor, such as chronic lymphocytic leukemia. In some embodiments, the hematologic tumor is a lymphoma, and the lymphoma expresses CD94/NKG2A.

In some embodiments, the autoimmune disease or inflammatory disease is selected from: polyarteritis nodosa, systemic lupus erythematosus, Wegener's granulomatosis, autoimmune hepatitis, Behcet's disease, Crohn's disease, primary biliary cirrhosis, scleroderma, ulcerative colitis, Sjögren's syndrome, type I diabetes, uveitis, Graves' disease, thyroiditis, type 1 diabetes mellitus, myocarditis, rheumatic fever, systemic sclerosis, ankylosing spondylitis, rheumatoid arthritis, glomerulonephritis, sarcoidosis, dermatomyositis, myasthenia gravis, polymyositis, Guillain-Barré syndrome, multiple sclerosis, alopecia areata, pemphigus/pemphigoid, psoriasis, and vitiligo.

In a seventh aspect, the present disclosure provides a method for preventing and/or treating a disease associated with abnormal expression or abnormal function of NKG2A, the method comprises administering to the human individual an effective amount of the anti-NGK2A antibody or the antigen-binding fragment thereof as described in any of the preceding items, or the biological material as described above, or the composition as described above.

In a eighth aspect, the present disclosure provides a method for enhancing the killing activity of NK92 cells or primary NK cells, the method comprises administering to the human individual an effective amount of the anti-NGK2A antibody or the antigen-binding fragment thereof as described in any of the preceding items, or the biological material as described above, or the composition as described above.

In a ninth aspect, the present disclosure provides a method for stimulating an immune response in a human individual, the method comprises administering to the human individual an effective amount of the anti-NGK2A antibody or the antigen-binding fragment thereof as described in any of the preceding items, or the biological material as described above, or the composition as described above. In some embodiments, the human individual suffers from a tumor and the anti-tumor immune response is stimulated. In other embodiments, the human individual suffers from a chronic viral infection and the anti-viral immune response is stimulated.

The anti-NGK2A antibody or antigen-binding fragment thereof, or the biological material described in the present disclosure, or the composition as described hereinbefore can be used as a medicament. In some embodiments, it is used as a medicament for preventing and/or treating diseases associated with abnormal expression or abnormal function of NKG2A. In some embodiments, it is used as a medicament for enhancing the killing activity of NK92 cells or primary NK cells. In some embodiments, it is used as a medicament for stimulating an immune response in a human individual.

It should be understood that within the scope of the present disclosure, the above-mentioned various technical features of the present disclosure and the various technical features described in detail below (such as in the Examples) can be combined with each other to form a new or preferred technical solution. Due to the limitations of length, no more tautology herein.

### Definition of terms

In the present disclosure, unless otherwise specified, the scientific and technical terms used herein have the meanings commonly understood by those skilled in the art. Moreover, the cell culture, biochemistry, nucleic acid chemistry, immunology laboratory and other operational steps used herein are all conventional steps widely used in the corresponding fields.

### NKG2A

Natural killer (NK) cells are a very important class of lymphocytes in the body, playing an important role in both natural and acquired immunity. There are two types of surface receptors on the surface of NK cells, which can be divided into inhibitory and activating receptors according to their functions, mediating different recognition modes of NK cells and transmitting different activation signals and inhibitory signals, respectively. The CD94/NKG2 family is a type of receptor family that has been studied more, mainly including NKG2A, NKG2B, NKG2C, NKG2D, NKG2E, NKG2F, NKG2H and other members. Among them, NKG2A is an inhibitory receptor whose ligand is the non-classical major histocompatibility complex class I molecule, HLA-E. HLA-E molecule expressed on target cells has an inhibitory effect on the killing function of NK cells after binding to NKG2A. Therefore, antibodies that inhibit CD94/NKG2A may increase the killing activity of tumor-specific lymphocytes against tumor cells. In addition, some lymphomas, such as NK-lymphomas, are characterized by CD94/NKG2A expression. In such patients, therapeutic antibodies that target and kill CD94/NKG2A-expressing cells may be able to eradicate tumor cells.

### Antibody

As used herein, natural "antibodies" refer to immune proteins that are produced by the body in response to antigen stimulation, have protective effects, are capable of binding to antigens, and are secreted by plasma cells (effector B cells). "Immunoglobulins" refer to globulins that have the activity or chemical structure of an antibody (Ab) and are similar to antibody molecules. Antibodies are usually composed of two pairs of polypeptide chains, each pair having one light chain (LC) and one heavy chain (HC). Light chains of antibodies can be classified into kappa and lambda light chains. Heavy chains are classified into five categories: µ, γ, α, δ and ε according to the different antigenicity of their constant regions, and the corresponding immunoglobulins are IgM, IgG, IgA, IgD and IgE, respectively. The isotypes of antibodies are defined as IgM, IgD, IgG, IgA and IgE, respectively. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain, CL. The constant domains are not directly involved in the binding of antibodies to antigens, but exhibit a variety of effector functions, such as mediating the binding of immunoglobulins to host tissues or factors, including the binding of various cells (e.g., effector cells) of the immune system and the first component (C1q) of the classical complement system. Within the light and heavy chains, the variable and constant regions are connected by a "J" region of about 12 or more amino acids, and the heavy chain further contains a "D" region of about 3 or more amino acids. The VH and VL regions can be further subdivided into regions with hypervariability (called complementarity determining regions (CDRs)), interspersed with more conserved regions called framework regions (FRs). Each VH and VL are composed of three CDRs and four FRs arranged from the amino terminus to the carboxyl terminus in the following order: FR1, CDR1, FR2, CDR2, FR3, CDR3, FR4. The variable regions (VH and VL) of each heavy/light chain pair form an antigen binding site, respectively. The distribution of amino acids in various regions or domains may follow the definition of Kabat, Sequences of Proteins of Immunological Interest (National Institutes of Health, Bethesda, Md. (1987 and 1991)), or Chothia & Lesk (1987) J. Mol. Biol. 196:901-917; Chothia et al., (1989) Nature 342:878-883.

As used herein, the term "complementarity determining region" or "CDR" refers to the amino acid residues in the variable regions of an antibody that are responsible for binding to the antigen. The variable regions of the heavy and light chains each contain three CDRs, named CDR1, CDR2 and CDR3. The precise boundaries of these CDRs may be defined according to various numbering systems known in the art, for example, as defined in the Kabat numbering system (Kabat et al., Sequences of Proteins of Immunological Interest, 5th Ed. Public Health Service, National Institutes of Health, Bethesda, Md, 1991), the Chothia numbering system (Chothia & Lesk (1987) J. Mol. Biol. 196: 901-917; Chothia et al., (1989) Nature 342: 878-883) or the IMGT numbering system (Lefranc et al., Dev. Comparat. Immunol. 27: 55-77, 2003). For a given antibody, a person skilled in the art will readily identify the CDRs defined by the various numbering systems. Furthermore, the correspondence between different numbering systems is well known to those skilled in the art (eg, see Lefranc et al., Dev. Comparat. Immunol. 27:55-77, 2003). The numbering conventions of the present disclosure are shown in Table 1 below.

**Table 1. Relationships between CDR numbering systems**

| CDR | IMGT | Kabat | Chothia |
|---|---|---|---|
| HCDR1 | 27-38 | 31-35 | 26-32 |
| HCDR2 | 56-65 | 50-65 | 52-56 |
| HCDR3 | 105-117 | 95-102 | 95-102 |
| LCDR1 | 27-38 | 24-34 | 24-34 |
| LCDR2 | 56-65 | 50-56 | 50-56 |
| LCDR3 | 105-117 | 89-97 | 89-97 |

Unless otherwise specified, the variable regions and CDR sequences in the examples of the present disclosure are all subject to the "Kabat" numbering convention. Although in specific embodiments, a numbering system (such as Kabat) is used to define amino acid residues, the corresponding technical solutions of other numbering systems will be regarded as equivalent technical solutions.

As used herein, the term "framework region" or "FR" residues refers to those amino acid residues in the variable region of an antibody other than the CDR residues defined above. The term "antibody" is not limited to any particular method of producing antibodies. For example, it includes recombinant antibodies, monoclonal antibodies and polyclonal antibodies. Antibodies may be antibodies of different isotypes, for example, IgG (e.g., IgG1, IgG2, IgG3 or IgG4 subtypes), IgA1, IgA2, IgD, IgE or IgM antibodies.

As used herein, the term "antigen-binding fragment" of an antibody refers to a polypeptide comprising a fragment of a full-length antibody, which retains the ability to specifically bind to the same antigen bound by the full-length antibody and/or competes with the full-length antibody for specific binding to the antigen, and which is also referred to as an "antigen-binding portion". See generally, Fundamental Immunology, Ch. 7 (Paul, W., ed., 2nd ed., Raven Press, N. Y. (1989), which is incorporated herein by reference in its entirety for all purposes. Antigen-binding fragments of antibodies may be produced by recombinant DNA techniques or by enzymatic or chemical cleavage of intact antibodies. Non-limiting examples of antigen-binding fragments include Fab, Fab', F(ab')₂, Fd, Fv, complementarity determining region (CDR) fragments, scFv, diabodies, single domain antibodies, chimeric antibodies, linear antibodies, nanobodies (technology from Domantis), and polypeptides that contain at least a portion of an antibody sufficient to confer specific antigen binding ability to the polypeptide. Engineered antibody variants are reviewed in Holliger et al., 2005; Nat Biotechnol, 23: 1126-1136.

The "Fab" is composed of one light chain and the CH1 and the variable region of one heavy chain.

The "Fab'" is a Fab fragment with one or more cysteine residues at the C-terminus of the CH1 domain. The "F(ab')₂" is a bivalent fragment formed by two Fab' fragments connected via disulfide bridges in the hinge region.

The "Fab'-SH" refers to a Fab' with a free thiol group on the cysteine residue of the constant domains.

The "single-chain Fab" is a polypeptide composed of an antibody heavy chain variable domain (VH), an antibody constant domain 1 (CH1), an antibody light chain variable domain (VL), an antibody light chain constant domain (CL) and a linker, wherein the antibody domains and the linker have one of the following orders from the N-terminus to the C-terminus: a) VH-CH1-linker-VL-CL, b) VL-CL-linker-VH-CH1, c) VH-CL-linker-VL-CH1, or d) VL-CH1-linker-VH-CL; wherein the linker is a peptide linker. In some embodiments, the peptide linker is a polypeptide of at least 30 amino acids, preferably a polypeptide of 32-50 amino acids.

The "diabodies" have two antigen binding sites, including a heavy chain variable domain (VH) and a light chain variable domain (VL) connected to each other in the same polypeptide chain.

The "linear antibody" contains a pair of tandem Fd fragments (VH-CH1-VH-CH1), which together with complementarity light chain polypeptides form a pair of antigen binding regions.

The "Fv" has the VL and VH domains of one arm of an antibody.

The "single domain antibody" is an antibody fragment that contains all or part of the heavy chain variable domain or all or part of the light chain variable domain of an antibody.

Single chain antibody fragment (scFv) is an antibody formed by a heavy chain variable region and a light chain variable region joined via a linker.

The term "Fc region" or "fragment crystallizable region" is used to define the C-terminal region of the heavy chain of an antibody, including a natural Fc region and a modified Fc region. In some embodiments, the Fc region contains two subunits with identical or different sequences. In some embodiments, the Fc region of a human IgG heavy chain is defined as extending from the amino acid residue at position Cys226 or from Pro230 to its carboxyl terminus. Suitable natural sequence Fc regions for antibodies described herein include human IgG1, IgG2 (IgG2A, IgG2B), IgG3, and IgG4. Unless otherwise indicated, the numbering convention for Fc regions is the EU index.

The term "IgG antibody" refers to an antibody having "heavy" chains and "light" chains cross-linked via intra-chain disulfide bonds and inter-chain disulfide bonds, with a typical "Y" structure. Each heavy chain consists of an N-terminal HCVR and a heavy chain constant region ("HCCR"). Each light chain consists of a LCVR and a light chain constant region ("LCCR").

As used herein, the term "full-length antibody" means an antibody consisting of two "full-length heavy chains" and two "full-length light chains". Wherein, "full-length heavy chain" refers to a polypeptide chain which, in the direction from N-terminus to C-terminus, is composed of a heavy chain variable region (VH), a heavy chain constant region CH1 domain, a hinge region (HR), a heavy chain constant region CH2 domain, and a heavy chain constant region CH3 domain; and, when the full-length antibody is an IgE isotype, optionally further comprises a heavy chain constant region CH4 domain. Preferably, the "full-length heavy chain" is a polypeptide chain composed of VH, CH1, HR, CH2 and CH3 in the direction from N-terminal to C-terminal. The "full-length light chain" is a polypeptide chain composed of a light chain variable region (VL) and a light chain constant region (CL) in the direction from N-terminal to C-terminal. Two pairs of full-length antibody chains are linked together by disulfide bonds between CL and CH1 and disulfide bonds between the HRs of two full-length heavy chains. The full-length antibody of the present disclosure may be from a single species, such as human; or may be a chimeric antibody or a humanized antibody. The full-length antibody of the present disclosure comprises two antigen-binding sites formed by the pairs of VH and VL, respectively, and the two antigen-binding sites specifically recognize/bind to the same antigen.

The term "capable of specifically binding", "specific binding" or "binding" means that an antibody is able to bind to an antigen or epitope with a higher affinity than other antigens or epitopes. Typically, the antibody binds to the antigen or epitope with an equilibrium dissociation constant (KD) of about 1×10⁻⁷ M or less (e.g., about 1×10⁻⁸ M or less). In some embodiments, the antibody binds to the antigen with a KD that is 10% or less (eg, 1%) of the KD of this antibody binding to a non-specific antigen (eg, BSA, casein). The KD may be measured using known methods, such as those measured by FACS or surface plasmon resonance assays. However, an antibody that specifically binds to an antigen or an epitope thereof may have cross-reactivity to other related antigens, for example, to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

As used herein, the terms "monoclonal antibody" and "mAb" have the same meaning and are used interchangeably, and refer to an antibody or a fragment of an antibody from a group of highly homologous antibody molecules, that is, a group of identical antibody molecules, except for natural mutations that may occur spontaneously. Monoclonal antibodies have high specificity for a single epitope on an antigen. Polyclonal antibodies are the opposite of monoclonal antibodies, typically comprising at least two or more different antibodies, and these different antibodies generally recognize different epitopes on an antigen. In addition, the modifier "monoclonal" only indicates that the antibody is characterized as being obtained from a highly homologous group of antibodies and is not to be understood as requiring any specific method for the preparation of said antibody.

The monoclonal antibodies of the present disclosure may be prepared by a variety of techniques, such as hybridoma technology (see, for example, Kohler et al., Nature, 256:495, 1975), recombinant DNA technology (see, for example, U.S. Patent Application 4,816,567), or phage antibody library technology (see, for example, Clackson et al., Nature 352: 624-628, 1991, or Marks et al., J. Mol. Biol. 222: 581-597, 1991).

Antibodies may be purified by well-known techniques, such as affinity chromatography using protein A or protein G. Subsequently or alternatively, the specific antigen (the target molecule recognized by the antibody) or its antigenic epitope may be fixed on a column and the immunospecific antibody may be purified by immunoaffinity chromatography. Purification of immunoglobulins may be referred to, for example, D. Wilkinson (The Scientist, published by The Scientist, Inc, Philadelphia Pa., Vol. 14, No. 8 (Apr. 17, 2000), pp. 25-28).

As used herein, the term "chimeric antibody" refers to an antibody in which a portion of the light chain or/and the heavy chain is derived from one antibody (which may be derived from a particular species or belong to a particular antibody class or subclass), and another portion of the light chain or/and the heavy chain is derived from another antibody (which may be derived from the same or different species or belong to the same or different antibody class or subclass), but in any case, it still retains binding activity to the target antigen (U.S.P 4, 816, 567 to Cabilly et al.; Morrison et al., Proc. Natl. Acad. Sci. USA, 81:6851 6855 (1984)). In certain embodiments, the term "chimeric antibody" may include antibodies (e.g., human-mouse combined antibodies) in which the heavy and light chain variable regions of the antibody are derived from a first antibody (e.g., a murine antibody), and the heavy and light chain constant regions of the antibody are derived from a second antibody (e.g., a human antibody).

As used herein, the term "humanized antibody" refers to a non-human antibody that has been genetically engineered, and whose amino acid sequence is modified to improve the homology with the sequence of a human antibody. Generally speaking, all or part of the CDR regions of a humanized antibody are derived from a non-human antibody (donor antibody), and all or part of the non-CDR regions (e.g., variable region FRs and/or constant regions) are derived from a human immunoglobulin (acceptor antibody). Typically, at least one or two, but usually all three, acceptor CDRs (of the heavy and/or light immunoglobulin chains) of a humanized antibody are replaced by donor CDRs. The immunoglobulin providing the CDRs is referred to as a "donor", and the immunoglobulin providing the frameworks is referred to as a "acceptor". In one embodiment, the donor immunoglobulin is a non-human (e.g., mouse) antibody, and the acceptor framework may be a naturally occurring human framework, or a sequence having about 85%, 90%, 95%, 99% or higher identity compared thereto. The humanized antibody generally retains the desired properties of the donor antibody, including but not limited to, antigen specificity, affinity, reactivity, etc. The donor antibody may be a mouse, rat, rabbit, or non-human primate (e.g., cynomolgus monkey) antibody with the desired properties (e.g., antigen specificity, affinity, reactivity, etc.).

The term sequence "identity" refers to the degree (percentage) to which the amino acids/nucleic acids of the two sequences are identical at equivalent positions when the two sequences are optimally aligned. During the alignment process, gaps may be introduced if necessary to obtain the maximum percentage of sequence identity, but any conservative substitutions are not considered to constitute part of the sequence identity. To determine the percentage of sequence identity, alignment may be achieved by techniques known in the art, for example, using publicly available computer software such as BLAST, BLAST-2, ALIGN, ALIGN-2 or Megalign (DNASTAR) software. Those skilled in the art can determine parameters suitable for measuring alignment, including any algorithm required to achieve maximum alignment over the full length of the sequences being aligned.

The term "nucleic acid" is used interchangeably with the term "polynucleotide" herein and refers to deoxyribonucleotides or ribonucleotides and polymers thereof in single-stranded or double-stranded form. The term encompasses nucleic acids containing known nucleotide analogs or modified backbone residues or linkages, which are synthetic, naturally occurring, and non-naturally occurring, have similar binding properties to reference nucleic acids, and are metabolized in a manner similar to reference nucleotides. Examples of such analogs include, but are not limited to, phosphorothioates, phosphoramidates, methylphosphonates, chiral-methylphosphonates, 2-O-methyl ribonucleotides, peptide-nucleic acids (PNAs). "Isolated" nucleic acids refer to nucleic acid molecules that have been separated from the components of their natural environment. The isolated nucleic acid encoding the antigen-binding molecule refers to one or more nucleic acid molecules encoding heavy and light chains (or fragments thereof) of the antibody, including such one or more nucleic acid molecules in a single vector or separate vectors, and such one or more nucleic acid molecules present in one or more locations in a host cell. Unless otherwise indicated, a specific nucleic acid sequence also implicitly encompasses conservatively modified variants (e.g., degenerate codon substitutions) and complementarity sequences thereof as well as explicitly indicated sequences. Specifically, as described in detail below, degenerate codon substitutions may be obtained by generating sequences in which the third position of one or more selected (or all) codons is substituted with a degenerate base and/or a deoxyinosine residue.

The term "pharmaceutical composition" refers to a mixture containing one or more antigen-binding molecules or antibodies described herein and other chemical components, such as physiologically/pharmaceutically acceptable carriers and excipients.

The term "pharmaceutically acceptable carrier" refers to an ingredient in the pharmaceutical formulation that is different from the active ingredient and is non-toxic to the subject. Pharmaceutically acceptable carriers include, but are not limited to, buffers, excipients, stabilizers or preservatives.

As used herein, the term "vector" refers to a nucleic acid delivery vehicle into which a polynucleotide can be inserted. When a vector enables the expression of the protein encoded by the inserted polynucleotide, the vector is called an expression vector. The vector can be introduced into a host cell by transformation, transduction or transfection so that the genetic material elements carried are expressed in the host cell. Vectors are well known to those skilled in the art, and include but are not limited to: plasmids, phagemids, cosmids, artificial chromosomes, such as yeast artificial chromosomes (YAC), bacterial artificial chromosomes (BAC) or P1-derived artificial chromosome (PAC); bacteriophages such as lambda phage or M13 phage and animal viruses, etc. Animal viruses that can be used as vectors include but are not limited to retroviruses (including lentiviruses), adenoviruses, adeno-associated viruses, herpes viruses (such as herpes simplex virus), poxviruses, baculoviruses, papillomaviruses, papovavirus (such as SV40). A vector may contain a variety of elements that control expression, including but not limited to promoter sequences, transcription initiation sequences, enhancer sequences, selection elements and reporter genes. In addition, the vector may also contain an origin of replication.

As used herein, the term "host cell" refers to a cell that can be used to introduce a vector, including but not limited to prokaryotic cells such as *Escherichia coli* or *Bacillus subtilis,* fungal cells such as yeast cells or *Aspergillus,* insect cells such as S2 Drosophila cells or Sf9, or animal cells such as fibroblasts, CHO cells, COS cells, NSO cells, HeLa cells, BHK cells, HEK293 cells or human cells.

The term "subject" or "individual" includes human and non-human animals. Non-human animals include all vertebrates (e.g., mammals and non-mammals) such as non-human primates (e.g., cynomolgus), sheep, dogs, cows, chickens, amphibians and reptiles. Unless explicitly stated, the terms "patient" or "subject" are used interchangeably herein. As used herein, the term "cyno" or "cynomolgus" refers to Macaca fascicularis. In certain embodiments, the individual or the subject is a human.

"Administration" or "administrating", when applied to an animal, human, experimental subject, cell, tissue, organ or biological fluid, refers to the contact of an exogenous drug, therapeutic agent, diagnostic agent or composition with an animal, human, subject, cell, tissue, organ or biological fluid.

The term "sample" refers to a collection such as fluid, cells, or tissue isolated from a subject, as well as fluid, cells or tissue present in a subject. Exemplary samples are biological fluids such as blood, serum and serosal fluid, plasma, lymph, urine, saliva, cystic fluid, tears, excretions, sputum, mucosal secretions of secretory tissues and organs, vaginal secretions, ascites, fluids of pleura, pericardium, peritoneum, abdominal cavity and other body cavities, fluids collected by bronchial lavage, synovial fluid, liquid solutions in contact with a subject or biological source, such as culture media for cell and organ (including conditioned media for cell and organ), lavage fluids, etc., tissue biopsy samples, fine needle aspirations, surgically removed tissues, organ cultures or cell cultures.

"Treatment or treat" and "treating or treated" refer to clinical interventions that are intended to be administered to the individual being treated, and may be performed for preventive purposes, or during the course of clinical pathology. The desired effects of treatment include, but are not limited to, preventing the occurrence or recurrence of the disease, alleviating symptoms, alleviating/reducing any direct or indirect pathological consequences of the disease, preventing metastasis, reducing the rate of disease progression, improving or alleviating the disease state, and regressing or improving prognosis. In some embodiments, the molecules of the present disclosure are used to delay the development of a disease or slow down the progression of a disease.

An "effective amount" is generally an amount sufficient to reduce the severity and/or frequency of symptoms, to eliminate such symptoms and/or potential causes, to prevent the onset of symptoms and/or potential causes thereof and/or to improve or ameliorate the damage caused by or associated with a disease state. In some embodiments, the effective amount is a therapeutically effective amount or a prophylactically effective amount. The "therapeutically effective amount" is an amount sufficient to treat a disease state or symptom, particularly a state or symptom associated with the disease state, or otherwise prevent, hinder, delay or reverse the disease state or the progression of any other undesirable symptom in any way associated with the disease. The "prophylactically effective amount" is an amount that, when administered to a subject, will have the intended prophylactic effect, such as preventing or delaying the onset (or recurrence) of the disease state, or reducing the likelihood of the onset (or recurrence) of the disease state or related symptoms. A full therapeutic or preventive effect may not occur after administering one dose, but may occur after administering a series of doses. Thus, a therapeutically or therapeutically effective amount may be administered in one or more administrations. The "therapeutically effective amount" and the "prophylactically effective amount" may vary depending on a variety of factors such as the disease state, age, sex, and weight of the individual, and the ability of the therapeutic agent or combination of therapeutic agents to elicit a desired response in the individual. Exemplary indicators of an effective therapeutic agent or combination of therapeutic agents include, for example, improved health condition of the patient.

HLA-E is a nonclassical MHC molecule that is expressed on the cell surface and is regulated by the binding of peptides derived from signal sequences of other MHC class I molecules. HLA-E binds natural killer (NK) cells and some T cells, and specifically binds to CD94/NKG2A, CD94/NKG2B, and CD94/NKG2C (see, e.g., Braud et al., (1998) Nature 391:795-799, which is incorporated herein by reference in its entirety). Surface expression of HLA-E is sufficient to protect target cells from lysis by CD94/NKG2A+NK, T, or NKT cell clones. As used herein, "HLA-E" refers to any variant, derivative, or isoform of the HLA-E gene or the encoded protein. Also included are any nucleic acid or protein sequences that share one or more biological properties or functions and share at least 70%, 80%, 90%, 95%, 96%, 97%, 98%, 99% or more nucleotide or amino acid identity with wild-type, full-length HLA-E. "NK" cells refer to a subpopulation of lymphocytes involved in non-traditional immunity. NK cells can be identified by certain characteristics and biological properties, such as the expression of specific surface antigens including CD16, CD56 and/or CD57, the lack of α/β or γ/δ TCR complexes on the cell surface, the ability to bind and kill cells that cannot express "self" MHC/HLA antigens by activating specific cytolytic enzymes, the ability to kill tumor cells or other disease cells that express ligands for NK-activated receptors, and the ability to release protein molecules (called cytokines) that stimulate or inhibit immune responses. Any of these characteristics or activities can be used to identify NK cells using methods well-known in the art.

### EFFECTS OF THE INVENTION

The antibody of the present disclosure can specifically bind to the NKG2A protein on the surface of NK cells as well as the soluble extracellular region protein of NKG2A, and significantly increase the killing effect of NK92 cells or primary NK cells on HLA-E positive tumor cells, and has a superior functional activity than known anti-NKG2A antibodies. The NKG2A antibody of the present disclosure has high specificity and has no cross-reaction with the human NKG2C family homologous protein antigens. Therefore, the antibody of the present disclosure (especially humanized antibody) have great clinical value.

### DESCRIPTION OF THE DRAWINGS

Figure 1 shows the results of ELISA detection of the binding reaction between NKG2A chimeric antibodies and the extracellular region of human NKG2A protein (hNKG2A/CD94-ECD-Fc).
Figure 2 shows the results of ELISA detection of the binding reaction between NKG2A chimeric antibodies and the extracellular region of cyno NKG2A protein (cynoNKG2A/CD94-ECD-Fc).
Figure 3 shows the results of FACS detection of the binding reaction between NKG2A chimeric antibodies and human NKG2A overexpressing cell line (CHOK1-hNKG2A/CD94).
Figure 4 shows the results of FACS detection of the binding reaction between NKG2A chimeric antibodies and cyno NKG2A overexpressing cell line (CHOK1-cynoNKG2A/CD94).
Figure 5 shows the results of FACS detection of the binding reaction between NKG2A chimeric antibodies and human NKG2C overexpressing cell line (CHOK1-hNKG2C/CD94).
Figure 6 shows the results of the activity detection of NKG2A chimeric antibodies in the experiment of mediating NK92 to kill LCL721.221 tumor cells (the antibodies enhanced the killing activity of NK92 cells).
Figure 7 shows the results of the activity detection of NKG2A chimeric antibodies in the experiment of mediating NK92 to kill MOLM-13 tumor cells (the antibodies enhanced the killing activity of NK92 cells).
Figure 8 shows the results of the activity detection (PBMC donor-1) of NKG2A chimeric antibodies in the experiment of mediating primary NK cells to kill LCL721.221 tumor cells. The antibodies enhanced the killing activity of primary NK cells.
Figures 9A~9E show the results of FACS detection of the binding reaction between humanized NKG2A antibodies and CHOK1-hNKG2A/CD94.
Figures 10A~10C show the results of FACS detection of the binding reaction between humanized NKG2A antibodies and CHOK1-cynoNKG2A/CD94.
Figure 11 shows the results of the activity detection of humanized NKG2A antibodies in the experiment of mediating NK92 to kill LCL721.221 tumor cells (antibodies enhanced the killing activity of NK92 cells).
Figures 12A~12D show the results of the activity detection (PBMC donor-2, donor-3, donor-4) of humanized NKG2A antibodies in the experiment of mediating primary NK cells to kill LCL721.221 tumor cells. The antibodies enhanced the killing activity of primary NK cells.

### DETAILED DESCRIPTION

The present disclosure will be described below in combination with specific examples, and the content of the present disclosure is not limited thereto.

Unless otherwise specified, the reagents and instruments used in the following methods are all reagents and instruments commonly used in the field and are commercially available, the methods used are conventional methods in the field, and those skilled in the art can undoubtedly implement the methods and obtain corresponding results based on the contents described in the examples.

Unless otherwise specified, the molecular biology experimental methods and immunoassays used in the present disclosure are basically carried out with reference to the methods described in J. Sambrook et al., Molecular Cloning: A Laboratory Manual, 2nd edition, Cold Spring Harbor Laboratory Press, 1989, and F.M. Ausubel et al., Short Protocols in Molecular Biology, 3rd edition, John Wiley & Sons, Inc., 1995. The restriction endonucleases are used in accordance with the conditions recommended by the product manufacturers. Those skilled in the art will appreciate that the Examples are used to describe the present disclosure by way of examples and are not intended to limit the scope of protection claimed by the present disclosure.

The room temperature described in the Examples is the conventional room temperature in the field, generally 10°C to 30°C.

Unless otherwise specified, the proteins, cells and mice used in the following Examples are all provided by Shanghai ChemPartner Co., Ltd.

In the following Examples, the antibodies constructed have a constant region of human IgG4 antibody. Those skilled in the art are aware of and able to obtain the sequence of the constant region of human IgG4 antibody.

### Example 1: Production of anti-NKG2A antibodies

The present disclosure uses hybridoma technology to produce monoclonal antibodies (mAbs) with specificity and high affinity for human and cyno NKG2A, and the antibodies do not bind to human NKG2C overexpressing cells.

14 groups of mice (including 4 groups of Balb/c mice, 9 groups of SJL mice and 1 group of NOD mice, all female mice aged 6 to 8 weeks) were immunized with the following immunogens: 293F-hNKG2A/CD94 stable cell line, recombinant hNKG2A/CD94-ECD-Fc protein, 293F-cynoNKG2A/CD94 stable cells + recombinant hNKG2A/CD94-ECD-Fc protein, hNKG2A/CD94 expression plasmid + recombinant hNKG2A/CD94-ECD-Fc protein. After immunization, the mice were tested for serum immune titer by FACS and ELISA. Wherein, CHOK1-hNKG2A/CD94, CHOK1-cynoNKG2A/CD94 and CHOK1-hNKG2C/CD94 stable cells were used for FACS detection, and CHOK1-blank cells were used as controls. hNKG2A/CD94-ECD-Fc protein and cynoNKG2A/CD94-ECD-Fc protein were used for ELISA detection. Mice with high immune titer were selected, spleen cells were taken and fused with myeloma cells, and two rounds of screening were performed using the FACS method. After the primary screening, the positive hybridoma clones were transferred to 24-well plates for culture, followed by a secondary screening. The positive clones obtained by screening were subcloned, and the subclones were tested again by the same method of primary and secondary screening methods. The positive monoclones were selected for antibody production, frozen storage and sequencing. A total of 52 positive monoclones were obtained after fusion of 12 hybridomas in this project.

### Example 2: Sequencing, production and purification of chimeric antibodies

The subcloned cells collected in Example 1 were frozen and sent to Biointron for determining and analyzing the gene sequences of the variable region of light and heavy chains of the antibody. After sequencing, 8 sequence-specific monoclonal antibodies were obtained, and the numbering of the monoclones as well as the names of the chimeric antibodies produced, and their light and heavy chain variable regions and CDR sequences are shown in Table 2 below.

**Table 2: Antibody numbers and their amino acid sequence numbers**

| Antibody number | Name of corresponding chimeric antibody | Heavy chain SEQ ID NO. | | | | Light chain SEQ ID NO. | | | |
|---|---|---|---|---|---|---|---|---|---|
| | | Variable region | VH-CDR1 | VH-CDR2 | VH-CDR3 | Variable region | VL-CDR1 | VL-CDR2 | VL-CDR3 |
| 9 | cAb-9 | 1 | 2 | 3 | 4 | 5 | 6 | 7 | 8 |
| 14 | cAb-14 | 9 | 10 | 11 | 12 | 13 | 14 | 15 | 16 |
| 17 | cAb-17 | 17 | 18 | 19 | 20 | 21 | 22 | 23 | 24 |
| 24 | cAb-24 | 25 | 26 | 27 | 28 | 29 | 30 | 31 | 32 |
| 33 | cAb-33 | 33 | 34 | 35 | 36 | 37 | 38 | 39 | 40 |
| 35 | cAb-35 | 41 | 42 | 43 | 44 | 45 | 46 | 47 | 48 |
| 43 | cAb-43 | 49 | 50 | 51 | 52 | 53 | 54 | 55 | 56 |
| 52 | cAb-52 | 57 | 58 | 59 | 60 | 61 | 62 | 63 | 64 |

DNA recombination technology was used to link the genes of the light and heavy chain variable region of the monoclonal antibodies obtained by sequencing to the genes of the constant region of human IgG4 antibody, and the obtained gene was linked into the expression vector pcDNA3.4 using the technology of total gene synthesis, the mammalian cell CHOK1 (purchased from ATCC) was transfected to express the chimeric antibody, and anti-human NKG2A human-mouse chimeric antibody was isolated from the serum-free culture supernatant containing the target antibody by purification using protein A affinity chromatography. Wherein the amino acid sequence of the heavy chain constant region of the chimeric antibody is as set forth in SEQ ID NO: 95, and the amino acid sequence of the light chain constant region is as set forth in SEQ ID NO: 96.

At the same time, the NKG2A positive control antibody Z270 (Monalizumab jointly developed by Innate Pharma and AstraZeneca) was generated based on the humZ270 sequence disclosed by IMGT/2Dstructure-DB card for INN 10113. The sequence link is https://www.imgt.org/3Dstructure-DB/cgi/details.cgi?pdbcode=10113.

The nucleotide sequences of the heavy and light chain variable regions of the antibodies are shown in Table 3.

**Table 3: Number of nucleotide sequences of the heavy and light chain variable regions of the NKG2A chimeric antibodies**

| Antibody number | SEQ IN NO. of the polynucleotide of the heavy chain variable region | SEQ IN NO. of the polynucleotide of the light chain variable region |
|---|---|---|
| cAb-9 | 69 | 70 |
| cAb-14 | 71 | 72 |
| cAb-17 | 73 | 74 |
| cAb-24 | 75 | 76 |
| cAb-33 | 77 | 78 |
| cAb-35 | 79 | 80 |
| cAb-43 | 81 | 82 |
| cAb-52 | 83 | 84 |

### Example 3: Detecting the binding activity of chimeric antibodies to proteins by ELISA

Two antigen proteins, human NKG2A extracellular region protein (hNKG2A/CD94-ECD-Fc) and cyno NKG2A extracellular region protein (cynoNKG2A/CD94-ECD-Fc), were diluted with PBS to 1 µg/mL, and added to a ELISA microplate at 100 µL/well and incubated at 4°C overnight. The plate was blocked with ELISA blocking solution (PBS phosphate buffer containing 1% BSA, pH7.4, the percentage was by mass) at 37°C for two hours. The gradient diluted antibodies were added, with a total of 8 concentration points (initial concentration of 100 nM, 10-fold gradient dilution, including point 0), 100 µL/well, incubated at 37°C for 1 hour. The plate was washed 3 times, and the secondary antibody (purchased from Thermofisher), anti-human IgG (Fab specific)-HRP (1:5000 dilution), was added, 100 µL/well, incubated at 37°C for 1 hour. The plate was washed three times, TMB chromogen solution was added, 100 µL/well, incubated at 37°C for 10 minutes, and then 50 µL 1N hydrochloric acid was added to terminate the chromogenic reaction. The OD readings at 450 nm were read using a microplate reader.

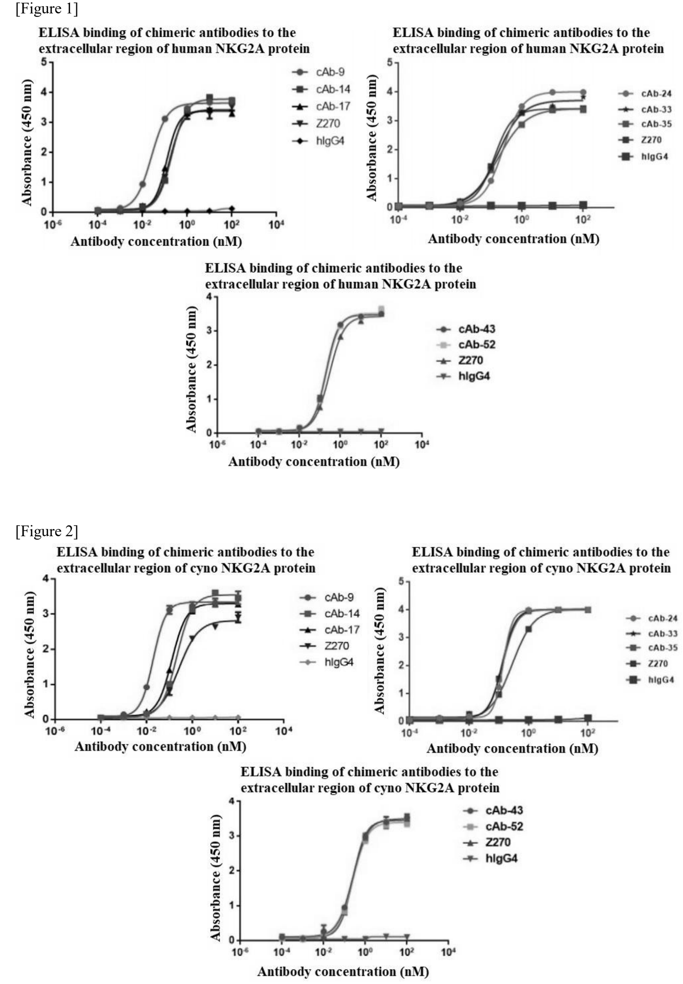

As shown in Figure 1, all the tested chimeric antibodies were bound to the hNKG2A/CD94-ECD Fc protein. As shown in Figure 2, all the tested chimeric antibodies were bound to the cynoNKG2A/CD94-ECD Fc protein.

### Example 4: Detecting the binding of chimeric antibodies to NKG2A/CD94 expressing cells by flow cytometry (FACS)

Human NKG2A overexpressing cells (CHOK1-hNKG2A/CD94), cyno NKG2A overexpressing cells (CHOK1-cynoNKG2A/CD94), and human NKG2C overexpressing cells (CHOK1-hNKG2C/CD94) were digested with TrypLE (purchased from Gibco). Cells were collected by centrifugation, then resuspended to 2E6/mL with FACS buffer (PBS+2%FBS). The cells were added to a 96-well cell plate at 100 µL per well, and the supernatant was discarded after centrifugation. The antibody was gradiently diluted with FACS buffer (initial concentration of 200 nM, 5-fold dilution, including point 0, a total of 10 concentration points), added to the cells at 100 µL/well, mixed, and incubated at 4°C for 1 hour. The incubated cells were centrifuged at 300 g for 5 min and washed twice with FACS buffer. Secondary antibody, anti-human IgG fluorescent secondary antibody 488 (purchased from Thermofisher A-11013), was added, incubated at 4°C for one hour, then the cells were washed twice with FACS buffer, resuspended in PBS and analyzed by FACS instrument.

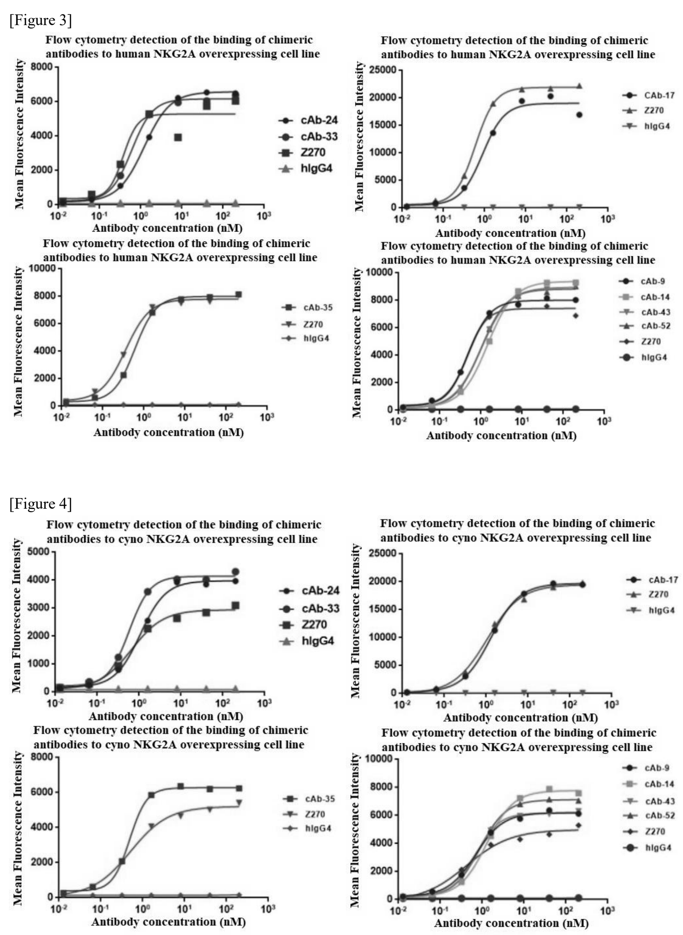

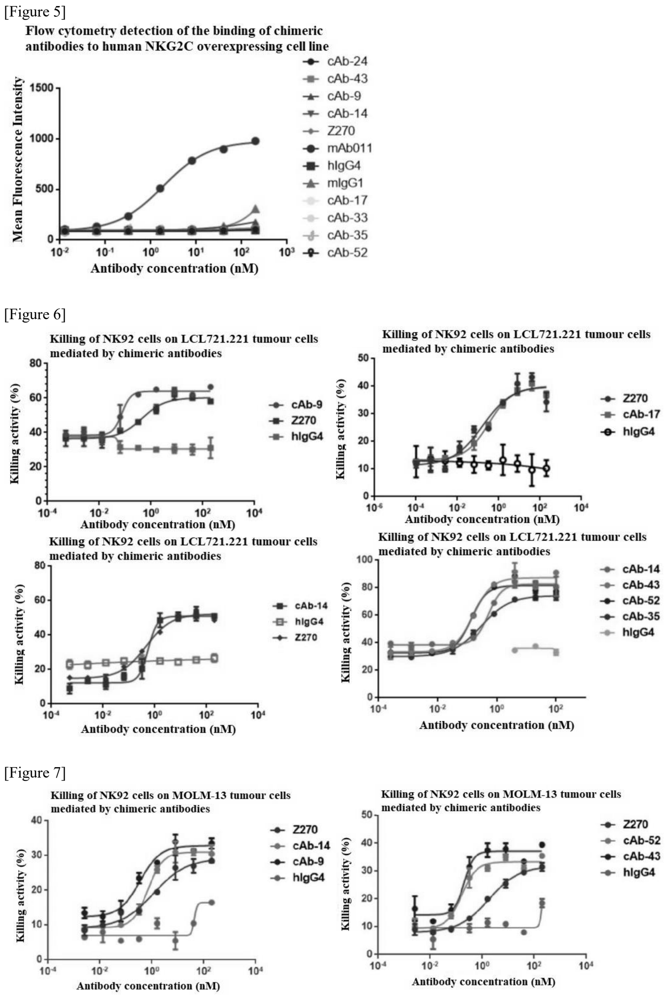

The experimental results are shown in Figures 3 and 4. The tested chimeric antibodies were all bound to CHOK1-hNKG2A/CD94 cells and CHOK1-cynoNKG2A/CD94 cells. As shown in Figure 5, none of the tested chimeric antibodies were bound to CHOK1-hNKG2C/CD94 cells, among which mAb011 was an anti-NKG2C positive control antibody (purchased from ChemPartner).

### Example 5: Anti-NKG2A antibody mediating the killing effect of NK92 cells on target cells

The target cells B lymphoblast LCL721.221 cells or human acute myeloid leukemia cells MOLM-13 cells (provided by Shanghai ChemPartner) were collected by centrifugation and resuspended to 2E6/mL in 1640 culture medium containing 10% fetal bovine serum. 0.5 mL of cell suspension was added to a 24-well plate, and the 24-well plate was placed in an incubator at 27°C overnight. NK92 cells (purchased from EK-Bioscience Biotechnology) were collected by centrifugation and washed twice with PBS and then counted by trypan blue staining, and the cell density was adjusted to 4E5/mL. 50 µL of NK92 cell suspension was added to a 96-well plate. The antibody to be tested was diluted with 1640 culture medium containing 10% fetal bovine serum, and 50 µL of the diluted antibody was added to the 96-well plate (initial concentration of 200 nM, 5-fold dilution, including point 0, a total of 10 concentration points). The culture plate was placed in an incubator at 37°C for 0.5 hours. Target cells were collected by centrifugation, washed twice with PBS, and resuspended to 1E6/mL in 1640 medium containing 10% fetal bovine serum. The marker BATDA (purchased from PerkinElmer) (2 µL/mL) was added to the target cell suspension, and the cells were placed in an incubator at 37°C for labeling for 20 minutes. The labeled target cells were washed 4 times by centrifugation and resuspended to 1E5/mL in 1640 medium containing 10% fetal bovine serum. 100 µL of the target cell suspension was added to a 96-well culture plate, and the culture plate was placed in an incubator at 37°C for 2 hours. The culture plate was centrifuged at 300 g for 5 min, 25 µL of the culture supernatant was transferred to another 96-well flat-bottom plate, and 200 µL of Eu-solution (purchased from PerkinElmer) was added. The data were read using the Envision instrument and analyzed using Graphpad software.

The experimental results are shown in Figure 6. In the experiment of NK92 cells killing LCL721.221 cells, all the tested chimeric antibodies had good activity in this experiment and could enhance the killing of NK92 on tumor cells, among which cAb-9, cAb-43 and cAb-52 were superior to the control antibody Z270. As shown in Figure 7, in the experiment of NK92 cells killing MOLM-13 cells, cAb-9, cAb-14, cAb-43, and cAb-52 all had strong activity and were superior to that of the control antibody Z270.

### Example 6: Detection of chimeric antibody-mediated killing effect of primary NK cells on target cells

Primary NK cells were isolated from PBMC (purchased from Milecell Biotechnologies) using an NK cell isolation kit and induced and cultured for 5-7 days, with a cell density of 2E6/mL. LCL721.221 cells were collected by centrifugation and resuspended to 2E6/mL in 1640 medium containing 10% fetal bovine serum (containing 1 mM inducing peptide). 0.5 mL of cell suspension was added to a 24-well plate, and the 24-well plate was placed in an incubator at 27°C overnight. NK cells were collected by centrifugation and washed twice with PBS and then counted by trypan blue staining, and the cell density was adjusted to 4E5/mL. 50 µL of cell suspension was added to a 96-well plate. The antibody to be tested was diluted with 1640 culture medium containing 10% fetal bovine serum, and 50 µL of the diluted antibody was added to the 96-well plate (initial concentration of 200 nM, 5-fold dilution, including point 0, a total of 10 concentration points). The culture plate was placed in an incubator at 37°C for 0.5 hours. LCL721.221 cells were collected by centrifugation, washed twice with PBS, and resuspended to 1E6/mL in 1640 medium containing 10% fetal bovine serum. The marker BATDA (purchased from PerkinElmer) (2 µL/mL) was added to the LCL721.221 cell suspension, and the cells were placed in an incubator at 37°C for labeling for 20 minutes. The LCL721.221 cells were washed 4 times by centrifugation and resuspended to 1E5/mL in 1640 medium containing 10% fetal bovine serum. 100 µL of the LCL721.221 cell suspension was added to a 96-well culture plate, and the culture plate was placed in an incubator at 37°C for 2 hours. The culture plate was centrifuged at 300 g for 5 min, 25 µL of the culture supernatant was transferred to another 96-well flat-bottom plate, and 200 µL of Eu-solution was added. The data were read using the Envision instrument and analyzed using Graphpad software.

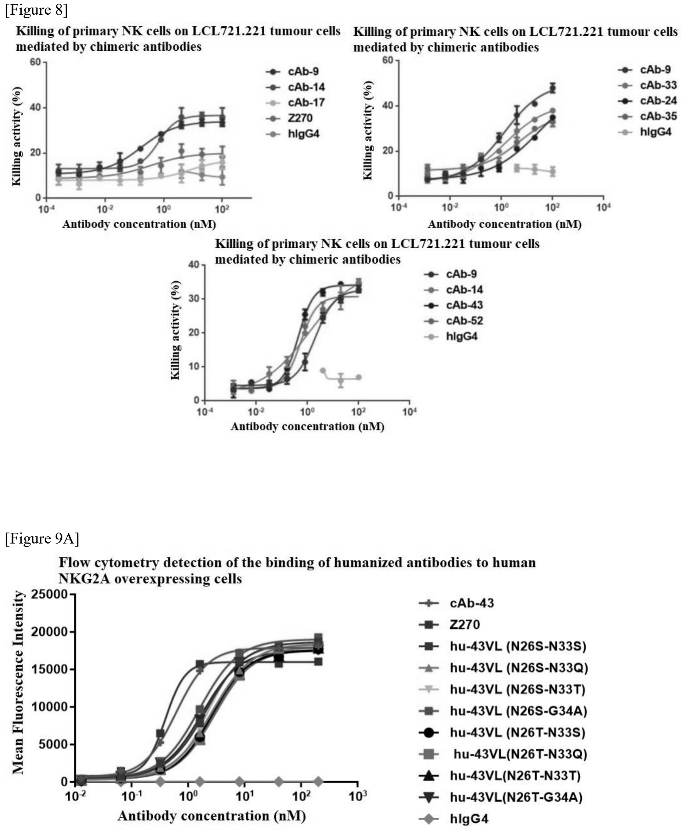

The experimental results are shown in Figure 8. Among the antibodies tested, cAb-17 had no activity in mediating primary NK cells to kill LCL721.221 cells, while the other antibodies had good activity in this experiment. cAb-9 and cAb-14 were significantly superior to the control antibody Z270, the other antibodies were compared with cAb-9, and cAb-43 and cAb-52 were superior to cAb-9.

### Example 7: Preparation of humanized antibodies

Monoclonal antibody No. 43 and monoclonal antibody No. 52 were selected for humanization, and the humanization design was completed by Biointron, that is, the humanization was performed by CDR grafting. The CDR regions of the molecule No. 43 or the antibody No. 52 were grafted to the gene framework sequence of the matching human variable region, and the reverse mutation was designed to finally obtain the heavy and light chain variable regions of the hu-43 humanized antibody and the hu-52 humanized antibody. Wherein, the monoclonal antibody No. 43 was humanized using the templates of two heavy chain variable regions of human antibody and the templates of three light chain variable regions, thereby obtaining the heavy chain variable regions of the hu-43 humanized antibody with sequences as set forth in SEQ ID NOs: 65, 85 and 86, respectively, and the light chain variable regions with sequences as set forth in SEQ ID NOs: 66, 87 and 88, respectively. Monoclonal antibody No. 52 was humanized using the templates of three heavy chain variable regions of human antibody and the templates of three light chain variable regions, thereby obtaining the heavy chain variable regions of the hu-52 humanized antibody with sequences as set forth in SEQ ID NOs: 89, 90 and 91, respectively, and the light chain variable regions with sequences as set forth in SEQ ID NOs: 92, 93 and 94, respectively.

The obtained variable regions of the humanized antibodies were combined and ligated with the heavy chain constant region (such as the heavy chain constant region of IgG4 as set forth in SEQ ID NO: 95) and the light chain constant region (such as the light chain constant region of kappa as set forth in SEQ ID NO: 96) of human antibodies to obtain full-length humanized antibodies. Exemplarily, hu-43 humanized antibodies are such as hu-43VH-11/VL-21, hu-43VH-11/VL-11, hu-43VH-13/VL-11, hu-43VH-13/VL-21, hu-43VH-13/VL-31, hu-43VH-21/VL-11, hu-43VH-21/VL-21, hu-43VH-21/VL-31, and hu-43VH-11/VL-31. hu-52 humanized antibodies are such as hu-52VH-13/VL-11, hu-52VH-23/VL-11, hu-52VH-33/VL-11, hu-52VH-13/VL-21, hu-52VH-23/VL-21, hu-52VH-33/VL-21, hu-52VH-13/VL-31, hu-52VH-23/VL-31, and hu-52VH-33/VL-31.

The variable region sequences of the hu-43VH-11/VL-21 humanized antibodies were analyzed, and there were potential post-translational modification sites (hereinafter referred to as hotspot(s)) in the first CDR region of the light chain, including the glycosylation NKS site and the deamidation NG site. Therefore, the light chain N26 was mutated to T, S, Q, A, D, G, E, F, I, K, L, P, V, W, Y, R, or H, G34 was mutated to A, L, E, K, R, S, N, T, or Q, or N33 was mutated to Q, S, T (here, one of the two G34/N33 mutation sites was chosen), the heavy chain remained unchanged. The designed mutation sequences are named according to the hotspot mutation sites of the light chain, i.e. hu-43VL (N26X₁-N33X₂ (or G34X₃)), wherein the amino acid X₁ is T, S, Q, A, D, G, E, F, I, K, L, P, V, W, Y, R or H; X₂ is Q, S or T; X₃ is A, L, E, K, R, S, N, T or Q.

The amino acid sequence of the heavy chain variable region of the humanized hotspot mutation molecule is as set forth in SEQ ID NO: 65, and the sequences of CDR1, CDR2 and CDR3 of the heavy chain are as set forth in SEQ ID NOs: 50, 51 and 52, respectively; the amino acid sequence of the light chain variable region is as set forth in SEQ ID NO: 67, and the sequences of CDR1, CDR2 and CDR3 of the light chain are as set forth in SEQ ID NOs: 68, 55 and 56, respectively, wherein, in SEQ ID NOs: 67 and 68, X₁ is T, S, Q, A, D, G, E, F, I, K, L, P, V, W, Y, R or H; X₂ is N, Q, S or T; X₃ is G, A, L, E, K, R, S, N, T or Q; wherein, one and only one of the two conditions must exist: X₂ is N or X₃ is G. Exemplarily, the sequences of the antibodies obtained after mutation are shown in Table 4.

**Table 4: Number of variable region sequences of the humanized antibodies and the hotspot mutation molecules thereof**

| Antibody number | SEQ ID NO. of heavy chain sequence | | | | SEQ ID NO. of light chain sequence | | | |
|---|---|---|---|---|---|---|---|---|
| | Variable region | VH-CDR1 | VH-CDR2 | VH-CDR3 | Variable region | VL-CDR1 | VL-CDR2 | VL-CDR3 |
| hu-43VH-11/VL-21 | 65 | 50 | 51 | 52 | **66** | **54** | 55 | 56 |
| hu-43 VH-11/VL-21 hotspot mutation molecule | 65 | 50 | 51 | 52 | **67** | **68** | 55 | 56 |
| hu43-VL(N26S-N33S) | 65 | 50 | 51 | 52 | **97** | **98** | 55 | 56 |
| hu43-VL(N26S-N33Q) | 65 | 50 | 51 | 52 | **99** | **100** | 55 | 56 |
| hu43-VL(N26S-N33T) | 65 | 50 | 51 | 52 | **101** | **102** | 55 | 56 |
| hu43-VL(N26S-G34A) | 65 | 50 | 51 | 52 | **103** | **104** | 55 | 56 |
| hu43-VL(N26T-N33S) | 65 | 50 | 51 | 52 | **105** | **106** | 55 | 56 |
| hu43-VL(N26T-N33Q) | 65 | 50 | 51 | 52 | **107** | **108** | 55 | 56 |
| hu43-VL(N26T-N33T) | 65 | 50 | 51 | 52 | **109** | **110** | 55 | 56 |
| hu43-VL(N26T-G34A) | 65 | 50 | 51 | 52 | **111** | **112** | 55 | 56 |
| hu43-VL(N26A-G34A) | 65 | 50 | 51 | 52 | **113** | **114** | 55 | 56 |
| hu43-VL(N26D-G34A) | 65 | 50 | 51 | 52 | **115** | **116** | 55 | 56 |
| hu43-VL(N26E-G34A) | 65 | 50 | 51 | 52 | **117** | **118** | 55 | 56 |
| hu43-VL(N26F-G34A) | 65 | 50 | 51 | 52 | **119** | **120** | 55 | 56 |
| hu43-VL(N26G-G34A) | 65 | 50 | 51 | 52 | **121** | **122** | 55 | 56 |
| hu43-VL(N26H-G34A) | 65 | 50 | 51 | 52 | **123** | **124** | 55 | 56 |
| hu43-VL(N26I-G34A) | 65 | 50 | 51 | 52 | **125** | **126** | 55 | 56 |
| hu43-VL(N26K-G34A) | 65 | 50 | 51 | 52 | **127** | **128** | 55 | 56 |
| hu43-VL(N26L-G34A) | 65 | 50 | 51 | 52 | **129** | **130** | 55 | 56 |
| hu43-VL(N26P-G34A) | 65 | 50 | 51 | 52 | **131** | **132** | 55 | 56 |
| hu43-VL(N26Q-G34A) | 65 | 50 | 51 | 52 | **133** | **134** | 55 | 56 |
| hu43-VL(N26R-G34A) | 65 | 50 | 51 | 52 | **135** | **136** | 55 | 56 |
| hu43-VL(N26V-G34A) | 65 | 50 | 51 | 52 | **137** | **138** | 55 | 56 |
| hu43-VL(N26W-G34A) | 65 | 50 | 51 | 52 | **139** | **140** | 55 | 56 |
| hu43-VL(N26Y-G34A) | 65 | 50 | 51 | 52 | **141** | **142** | 55 | 56 |
| hu43-VL(N26S-G34E) | 65 | 50 | 51 | 52 | **143** | **144** | 55 | 56 |
| hu43-VL(N26S-G34G) | 65 | 50 | 51 | 52 | **145** | **146** | 55 | 56 |
| hu43-VL(N26S-G34K) | 65 | 50 | 51 | 52 | **147** | **148** | 55 | 56 |
| hu43-VL(N26S-G34L) | 65 | 50 | 51 | 52 | **149** | **150** | 55 | 56 |
| hu43-VL(N26S-G34N) | 65 | 50 | 51 | 52 | **151** | **152** | 55 | 56 |
| hu43-VL(N26S-G34Q) | 65 | 50 | 51 | 52 | **153** | **154** | 55 | 56 |
| hu43-VL(N26S-G34R) | 65 | 50 | 51 | 52 | **155** | **156** | 55 | 56 |
| hu43-VL(N26S-G34S) | 65 | 50 | 51 | 52 | **157** | **158** | 55 | 56 |
| hu43-VL(N26S-G34T) | 65 | 50 | 51 | 52 | **159** | **160** | 55 | 56 |

The amino acid sequences of multiple humanized hotspot mutant molecules were reverse translated into base sequences and cloned into the mammalian expression vector pCDNA3.4 to obtain recombinant expression vectors, which were then transfected into HEK293 or CHO-S cells by transient transfection. After 5 days of serum-free culture, the cell culture supernatant was harvested, and the antibody protein was harvested after affinity chromatography purification. The obtained hu-43VH-11/VL-21 mutant antibody comprised the heavy chain constant region of IgG4 as set forth in SEQ ID NO: 95 and the light chain constant region of kappa as set forth in SEQ ID NO: 96. Activity and function of multiple humanized hotspot mutant molecules of hu-43VH-11/VL-21 and Z270 were evaluated in vitro.

In order to further reduce the Fc-mediated ADCP activity and enhance the stability of the antibodies, the inventors mutated the heavy chain constant region of hu43-VL (N26S-G34A) and hu-52VH33/VL11 antibodies, with the mutation sites being L235E, D265A and R409K, and new antibody molecules hu43 (N26S-G34A) -IgG4 mutant antibody and hu-52VH33/VL11 -IgG4 mutant antibody were obtained.

### Example 8: Detection of activity and function of humanized antibodies in vitro

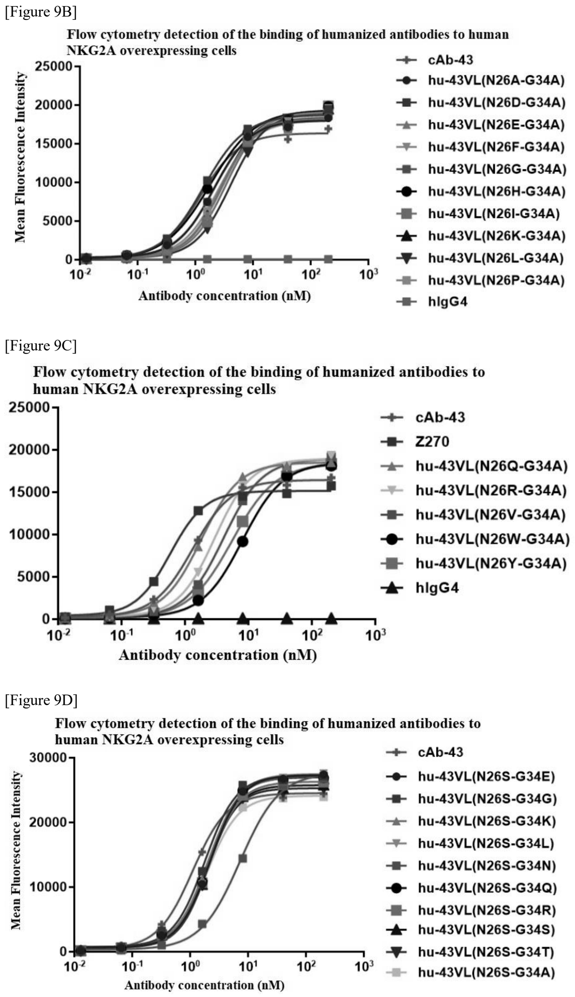

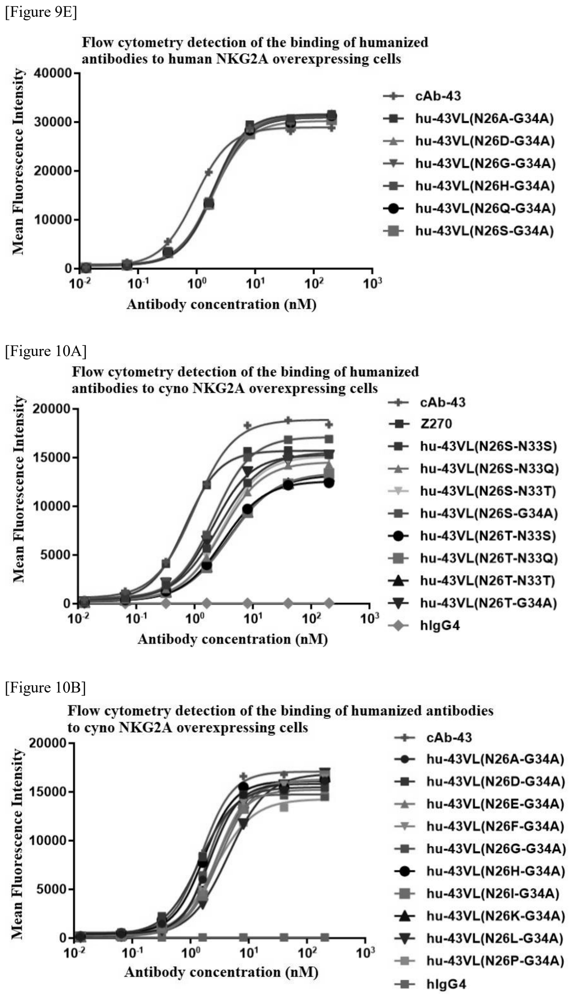

The binding activity of humanized antibodies to human NKG2A overexpressing cell line was detected by FACS (refer to Example 4 for FACS). The binding of hu-43VH-11/VL-21 hotspot mutant molecules, cAb-43 and control molecule Z270 to cells is shown in Figure 9 (A-E). The maximum MFIs of cAb-43 and its humanized mutant antibodies to human NKG2A overexpressing cell line were greater than that of the Z270 control antibody. The binding activities of hu-43VH-11/VL-21 hotspot mutant molecules were slightly decreased compared with that of cAb-43, but the activities of most molecules were comparable. The binding activities of hu-52 humanized antibodies are shown in Table 5. Table 5 shows that the maximum MFIs of hu-52 humanized antibodies are higher than that of the Z270 control antibody, indicating that the humanized hu-52 has better binding activity with the human NKG2A overexpressing cell line.

**Table 5. Binding activities of hu-52 humanized antibodies to human NKG2A overexpressing cell line**

| Antibody | Maximum MFI |
|---|---|
| hu-52VH-13/VL-11 | 18830 |
| hu-52VH-23/VL-11 | 18687 |
| hu-52VH-33/VL-11 | 18799 |
| hu-52VH-13/VL-21 | 18901 |
| hu-52VH-23/VL-21 | 18012 |
| hu-52VH-33/VL-21 | 18009 |
| hu-52VH-13/VL-31 | 18765 |
| hu-52VH-23/VL-31 | 18140 |
| cAb52 | 18762 |
| Z270 | 16093 |

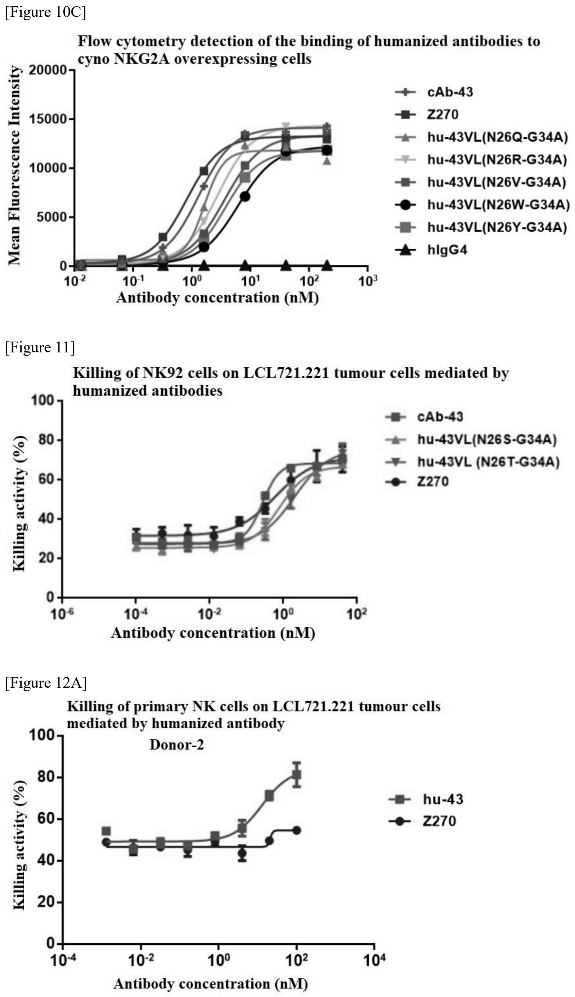

The binding activity of humanized antibodies to cyno NKG2A overexpressing cells was detected by FACS (refer to Example 4 for FACS). The binding of hu-43VH-11/VL-21 site-directed mutant molecules, cAb-43 and control molecule Z270 to cells is shown in Figure 10 (A-C). The binding activity of cAb-43 was comparable to that of the control molecule Z270, and the binding activities of the hu-43VH11/VL21 hotspot mutant molecules were slightly decreased, but the activities of most molecules were comparable. This indicates that multiple mutations at this amino acid site are all effective mutations.

The humanised antibodies mediated the killing effect of NK92 cells on LCL721.221 cells, with reference to Example 5. The results are shown in Figure 11, Tables 6-1 and 6-2. Tables 6-1 and 6-2 show that the humanized antibodies still retain a higher killing effect on target cells, and the killing activity is superior to that of Z270. Figure 11 shows that the killing activities of NK92 cells on target cells mediated by two hu-43VH-11/VL-21 humanized hotspot mutant molecules hu-43VL (N26S-G34A) and hu-43VL (N26T-G34A) are comparable, which are close to that mediated by Z270, and slightly weaker than the chimeric antibody cAb-43.

**Table 6-1. Killing results of hu-43 humanized antibodies on target cells**

| Antibody | IC50 (nM) |
|---|---|
| hu-43VH-13/VL-11 | 0.37 |
| hu-43VH-21/VL-11 | 0.52 |
| hu-43VH-11/VL-21 | 0.33 |
| hu-43VH-11/VL-31 | 0.45 |
| cAb-43 | 0.28 |
| Z270 | 1.11 |

**Table 6-2. Killing results of hu-52 humanized antibodies on target cells**

| Antibody | IC50 (nM) |
|---|---|
| hu-52VH-13/VL-11 | 0.19 |
| hu-52VH-23/VL-11 | 0.13 |
| hu-52VH-33/VL-11 | 0.09 |
| hu-52VH1-3/VL-21 | 0.14 |
| hu-52VH-23/VL-21 | 0.12 |
| hu-52VH-13/VL-31 | 0.16 |
| cAb-52 | 0.28 |
| Z270 | 0.57 |

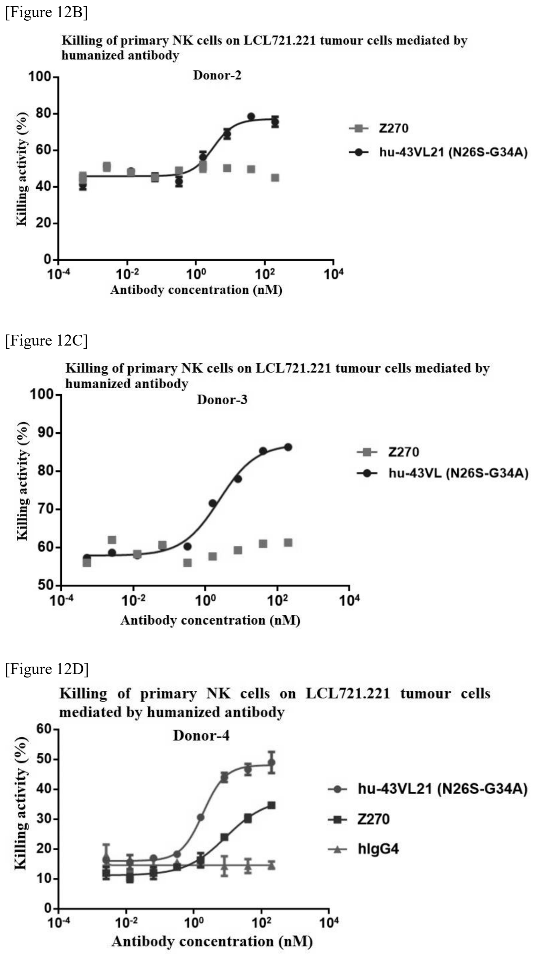

Humanized antibodies mediated primary NK cells to kill LCL721.221 cells, with reference to Example 6,. The results are shown in Figure 12 (A-D). Multiple experiments were performed to detect the killing effects of primary NK cells from different donors on LCL721.221 cells mediated by hu-43 and hotspot mutant molecule hu-43VL (N26S-G34A) and Z270, and all showed that the killing activity of hu-43VL (N26S-G34A) was significantly superior to that of the control molecule Z270.

Although the specific embodiments of the present disclosure have been described in detail, those skilled in the art will understand that various modifications and changes can be made to the details based on all the teachings that have been published, and these changes are all within the scope of protection of the present disclosure. All of the invention is given by the attached claims and any equivalents thereof.

### SEQUENCE LISTING

1: cAb-9 VH 
EVQLQQSVAELVRPGASVKLSCTASGFNIQNTYMHWVKERPEQGLEWIGRIDPANGDTEFAPKFQ
GKATLTTDTSSNTAYLQLSSLTSEDTAIYYCGRERYFYGSRGFAYWGQGTLVTVSA
2: cAb-9 VH-CDR1
   NTYMH
3: cAb-9 VH-CDR2
   RIDPANGDTEFAPKFQG
4: cAb-9 VH-CDR3
   ERYFYGSRGFAY
5: cAb-9 VL 
DILMTQSPPSMSVSLGDTVSITCHASQGISSNIGWLQQKPGKSFKGLIYHGTNLEDGVPSRFSGSGS
GADYSLTISSLESEDFADYYCVQYAQFPWTFGGGTKLEIK
6: cAb-9 VL-CDR1
   HASQGISSNIG
7: cAb-9 VL-CDR2
   HGTNLED
8: cAb-9 VL-CDR3
   VQYAQFPWT
9: cAb-14 VH 
EVQLQQSVAELVRPGASVKLSCTASGFNIKNTYIHWVKQRPEQGLEWIGRIDPANGNTKYAPKFQ
GKASIIVDTSSNTAYLQLSSLTSEDTAIYYCARYYASSPYYYNMDYWGQGTSVTVSS
10: cAb-14 VH-CDR1
   NTYIH
11: cAb-14 VH-CDR2
   RIDPANGNTKYAPKFQG
12: cAb-9 VH-CDR3
   YYASSPYYYNMDY
13: cAb-9 VL 
DIQMTQSPASLSVSVGETVTITCRASENIYSNLAWYQQKQGKSPQLLVYAATNLADGVPSRFSGSG
SGTQYSLKINSLQSEDFGSYYCQHFWGTFTFGSGTKLEIK
14: cAb-14 VL-CDR1
   RASENIYSNLA
15: cAb-14 VL-CDR2
   AATNLAD
16: cAb-14 VL-CDR3
   QHFWGTFT
17: cAb-17 VH 
EVQLQQSVAELVRPGASVKLSCTASGFNIKNTYIDWVKQRPEQGLEWIGNIDPANGNTKYAPKFQ
GKATITADTSSNTAYVQLSSLTSEDTAIYYCARWGGWDVRFAYWGQGTLVTVSA
18: cAb-17 VH-CDR1
   NTYID
19: cAb-17 VH-CDR2
   NIDPANGNTKYAPKFQG
20: cAb-17 VH-CDR3
   WGGWDVRFAY
21: cAb-17 VL 
DIKMTQSPSSMYASLGERVTITCKASQDINTYLSWFQQKPGKSPKTLIYRANRLVDGVPSRFSGSG
SGQNYSLTISSLEYEDMGIYYCLQYDEFPLTFGAGTKLELK
22: cAb-17 VL-CDR1
   KASQDINTYLS
23: cAb-17 VL-CDR2
   RANRLVD
24: cAb-17 VL-CDR3
   LQYDEFPLT
25: cAb-24 VH 
EVQLQQSVAELVRPGASVKLSCTASGFNIQNTYMHWVKQRPEQGLEWIGRIDPANGNTKYAPKFQ
GKATITADTSSNTAYLQLSSLTSEDTAIYYCARYYDSRYGYWGQGTTLTVSS
26: cAb-24 VH-CDR1
   NTYMH
27: cAb-24 VH-CDR2
   RIDPANGNTKYAPKFQG
28: cAb-24 VH-CDR3
   YYDSRYGY
29: cAb-24 VL 
DIKMTQSPSSMYASLGERVTITCKASQDINSYLSWFQQKPGKSPTTLIYRANRLVDGVPSRFSGSGS
GQDYSLTISSLEYEDLGIYFCLQYDEFPWTFGGGTKLEIK
30: cAb-24 VL-CDR1
   KASQDINSYLS
31: cAb-24 VL-CDR2
   RANRLVD
32: cAb-24 VL-CDR3
   LQYDEFPWT
33: cAb-33 VH 
EVQLQQSVAELVRPGASVKLSCTASGFNIKNTYMDWVKQRPEQGLEWIGRIDPANGNTKYAPKFQ
GKATITADTSSNTAYLQLSSLTSEDTAIYYCAREGNWDDWFVYWGQGTLVTVSA
34: cAb-33 VH-CDR1
   NTYMD
35: cAb-33 VH-CDR2
   RIDPANGNTKYAPKFQG
36: cAb-33 VH-CDR3
   EGNWDDWFVY
37: cAb-33 VL 
DVLMTQTPLSLPVSLGDQASISCRSSQSIVHSNGNTYLEWFLQKPGQSPKLLIYKVSNRFSGVPDR
FSGSGSGTDFTLKISRVEAEDLGVYYCFQGSHVPPTFGGGTKLEIK
38: cAb-33 VL-CDR1
   RSSQSIVHSNGNTYLE
39: cAb-33 VL-CDR2
   KVSNRFS
40: cAb-33 VL-CDR3
   FQGSHVPPT
41: cAb-35 VH 
EVQLQQSVAELVRPGASVKLSCTASGFNIKNTYMDWVKQRPEQGLEWIGRIDPANGNTKHAPKF
QGKATITTDTSSNTAYLQLSSLTSEDTAIYYCARWGGWDVRFAYWGQGTLVTVSA
42: cAb-35 VH-CDR1
   NTYMD
43: cAb-35 VH-CDR2
   RIDPANGNTKHAPKFQG
44: cAb-35 VH-CDR3
   WGGWDVRFAY
45: cAb-35 VL 
DIKMTQSPSSMYASLGERVTINCQASQDINSYLSWFQQKPGKSPKTLIHRANRLVDGVPSRFSGSG
SGQDYSLTISSLEYEDMGIYYCLQFDEFPLTFGAGTKLELK
46: cAb-35 VL-CDR1
   QASQDINSYLS
47: cAb-35 VL-CDR2
   RANRLVD
48: cAb-35 VL-CDR3
   LQFDEFPLT
49: cAb-43 VH 
EVQLQQSVAELVRPGASVKLSCTASGFNIQNTYIHWVKQRPEQGLEWIGRIDPASGSTEYAPKFQG
KATITADTSSNSGYLQLSSLTSEDTAIYYCARYGNFLYYYSMDYWGQGTSVTVSA
50: cAb-43 VH-CDR1
   NTYIH
51: cAb-43 VH-CDR2
   RIDPASGSTEYAPKFQG
52: cAb-43 VH-CDR3
   YGNFLYYYSMDY
53: cAb-43 VL 
DIVMTQAAPSVPVTPGESVSISCRSNKSLLHSNGNTYLYWFLQRPGQSPQLLIYRMSNLASGVPDR
FSGSGSGTAFTLRISRVEAEDVGVYYCMQHLENPYTFGGGTKLEIK
54: cAb-43 VL-CDR1
   RSNKSLLHSNGNTYLY
55: cAb-43 VL-CDR2
   RMSNLAS
56: cAb-43 VL-CDR3
   MQHLENPYT
57: cAb-52 VH 
EVQLQQSVAELVRPGASVKLSCTASGFNIQNTYLHWVKQRPEQGLEWIGRIDPANDNIKYVPKFQ
GKATITADTSSNTAYLQLSSLTSEDTAIYYCTRHGLFYEGWFAYWGQGTLVTVST
58: cAb-52 VH-CDR1
   NTYLH
59: cAb-52 VH-CDR2
   RIDPANDNIKYVPKFQG
60: cAb-52 VH-CDR3
   HGLFYEGWFAY
61: cAb-52 VL 
EIVLTQSPALMAASPGEKVTITCSVSSSISSSNLHWYQQKSGTSPKPWIYGTSNLASGVPVRFSGSG
SGTSYSLTISSMEAEDAATYYCQQWSSYPYTFGSGTKLEIK
62: cAb-52 VL-CDR1
   SVSSSISSSNLH
63: cAb-52 VL-CDR2
   GTSNLAS
64: cAb-52 VL-CDR3
   QQWSSYPYT
65: hu-43 VH-11 
QVQLVQSGAEVKKPGASVKVSCKASGFNIQNTYIHWVRQAPGQGLEWMGRIDPASGSTEYAPKF
OGRVTMTADTSTNTGYMELSSLRSEDTAVYYCARYGNFLYYYSMDYWGOGTLVTVSS
66: hu-43 VL-21 
DIVMTQSPLSLPVTLGQPASISCRSNKSLLHSNGNTYLYWFQQRPGQSPRLLIYRMSNLASGVPDR
FSGSGSGTDFTLKISRVEAEDVGVYYCMQHLENPYTFGQGTKLEIK
67: Light chain variable region of humanized hotspot mutant molecule 
DIVMTQSPLSLPVTLGQPASISCRSX1KSLLHSX2X3NTYLYWFQQRPGQSPRLLIYRMSNLASGVPD
RFSGSGSGTDFTLKISRVEAEDVGVYYCMQHLENPYTFGQGTKLEIK
68: VL-CDR1 of humanized hotspot mutant molecule
   RSX₁KSLLHSX₂X₃NTYLY
69: cAb-9 VH 
GAGGTACAGTTGCAACAGTCTGTTGCAGAGCTTGTGAGGCCAGGTGCCTCTGTCAAACTGTCT
TGTACTGCCAGCGGGTTCAATATCCAGAACACATATATGCACTGGGTAAAGGAGAGGCCAGAG
CAGGGCTTGGAGTGGATAGGAAGAATCGACCCAGCCAATGGCGACACTGAGTTTGCTCCTAA
GTTCCAGGGGAAAGCTACCCTCACAACAGATACCTCTAGCAACACTGCCTATCTCCAGCTGTC
CTCTCTGACCTCCGAGGATACTGCCATCTACTATTGCGGCAGAGAGAGATACTTCTACGGAAGC
AGAGGCTTCGCCTATTGGGGGCAAGGAACACTGGTTACAGTGTCTGCA
70: cAb-9 VL 
GACATATTGATGACACAGTCTCCTCCCTCTATGTCAGTCTCACTGGGCGATACTGTTTCAATTAC
ATGCCACGCCAGCCAGGGCATTTCATCAAACATCGGCTGGTTGCAGCAGAAACCAGGCAAGT
CTTTCAAGGGACTCATCTATCACGGCACCAACTTGGAGGATGGTGTGCCTTCCCGATTCTCCGG
ATCAGGCTCCGGCGCCGATTACTCCCTGACCATTTCTAGCCTGGAATCCGAGGACTTCGCCGA
CTACTATTGTGTACAGTATGCTCAGTTCCCATGGACATTCGGAGGGGGAACCAAGCTCGAAATA
AAA
71: cAb-14 VH 
GAAGTACAGCTTCAACAGTCAGTAGCCGAGTTGGTGAGACCAGGTGCATCAGTGAAGTTGAG
CTGTACTGCTAGTGGTTTCAACATTAAGAACACATATATCCACTGGGTCAAACAGAGGCCCGA
GCAAGGACTGGAGTGGATAGGCAGGATCGATCCAGCAAACGGAAATACCAAATACGCTCCTA
AGTTTCAGGGGAAGGCTTCCATTATTGTGGATACCAGTTCTAACACGGCCTACCTGCAGCTGTC
CAGCCTCACCTCCGAGGATACCGCCATCTACTACTGCGCCCGATATTATGCCTCCAGCCCCTATT
ATTATAATATGGATTACTGGGGCCAGGGCACTTCCGTTACAGTGAGCTCC
72: cAb-14 VL 
GATATTCAGATGACCCAGTCTCCGGCAAGCCTGTCAGTGAGTGTGGGCGAGACAGTGACAATC
ACCTGTAGGGCCAGCGAAAACATCTATAGCAATCTCGCATGGTATCAACAGAAACAGGGTAAG
TCACCACAGCTGCTTGTGTATGCTGCCACCAACCTGGCCGATGGAGTCCCATCCAGATTTTCTG
GCTCTGGGAGCGGGACACAATACTCCCTCAAGATTAACAGCCTCCAAAGCGAGGACTTTGGG
TCCTACTACTGCCAGCACTTTTGGGGAACATTTACGTTCGGCAGCGGGACAAAACTCGAAATC
AAG
73: cAb-17 VH 
GAAGTTCAGCTTCAACAGTCTGTGGCTGAACTTGTACGCCCAGGGGCATCCGTGAAGTTGAGT
TGTACAGCCAGCGGCTTCAATATCAAAAACACCTACATCGATTGGGTGAAACAGCGACCTGAG
CAGGGACTGGAATGGATCGGCAACATTGACCCGGCTAACGGCAACACCAAATACGCTCCGAA
ATTCCAGGGTAAAGCTACTATTACAGCTGATACCTCCTCCAACACCGCCTACGTCCAGCTGAGC
AGCCTGACCTCTGAAGATACAGCTATCTACTACTGTGCCAGGTGGGGAGGTTGGGACGTGAGG
TTCGCCTATTGGGGACAGGGAACCCTCGTCACTGTCAGCGCT
74: cAb-17 VL 
GACATCAAGATGACGCAGTCACCAAGCTCTATGTACGCCTCACTGGGGGAGCGAGTAACCATC
ACTTGTAAGGCATCCCAGGATATCAACACTTACCTGAGCTGGTTTCAGCAAAAGCCTGGCAAG
TCCCCCAAGACCCTCATCTATAGAGCTAATCGCCTCGTGGACGGAGTGCCCAGCCGGTTTAGT
GGATCCGGATCCGGCCAGAACTATTCCCTGACAATCAGCAGCCTGGAGTATGAGGACATGGGG
ATTTATTACTGTCTGCAATATGATGAGTTCCCGCTCACTTTCGGAGCCGGAACCAAGCTTGAGT
TGAAG
75: cAb-24 VH 
GAGGTACAGCTGCAGCAATCTGTTGCCGAATTGGTGAGACCAGGTGCCTCAGTGAAACTGTCT
TGCACTGCCTCTGGATTCAATATCCAGAATACTTACATGCATTGGGTCAAGCAGAGACCCGAAC
 
AGGGGCTCGAATGGATAGGAAGGATTGATCCCGCTAACGGCAACACAAAGTACGCTCCAAAA
TTCCAAGGAAAAGCCACTATAACCGCCGATACTAGCAGCAATACCGCTTATCTCCAGCTGTCTT
CCCTGACAAGTGAGGACACGGCCATTTATTATTGTGCCAGATATTACGATAGTAGGTACGGATAT
TGGGGACAGGGAACAACACTGACAGTGTCCTCC
76: cAb-24 VL 
GACATCAAGATGACACAGTCCCCATCTAGTATGTACGCTAGCCTTGGTGAGCGGGTGACAATC
ACTTGCAAAGCCTCCCAGGACATCAACTCATACCTGTCCTGGTTCCAGCAGAAGCCCGGGAA
GAGTCCAACCACACTGATATACCGAGCCAACCGACTGGTAGATGGCGTGCCTTCTCGGTTCTC
CGGCTCAGGTAGCGGGCAGGACTACTCCCTGACTATTTCCTCTCTCGAATACGAAGATCTGGG
CATCTACTTCTGTCTCCAGTACGACGAATTTCCCTGGACGTTCGGAGGAGGAACCAAGCTTGA
GATTAAG
77: cAb-33 VH 
GAGGTGCAGTTGCAGCAAAGCGTTGCAGAACTTGTTAGACCTGGTGCTAGTGTGAAACTGAG
CTGCACCGCCAGTGGATTTAACATCAAGAACACCTACATGGACTGGGTCAAACAGAGGCCCG
AGCAGGGGTTGGAGTGGATTGGACGAATTGATCCCGCAAACGGGAACACAAAATACGCTCCT
AAGTTTCAGGGAAAAGCCACCATTACAGCAGACACCAGTAGCAATACCGCTTATCTGCAGCTG
TCTAGCCTGACCAGCGAGGACACCGCAATATACTACTGCGCCAGAGAAGGGAATTGGGATGAT
TGGTTCGTATACTGGGGACAGGGCACACTGGTGACAGTCTCTGCT
78: cAb-33 VL 
GATGTCCTGATGACCCAGACCCCACTGAGTCTCCCAGTAAGTTTGGGCGACCAGGCATCAATC
TCATGTAGAAGCAGCCAGTCTATCGTGCACAGCAACGGCAATACATACCTCGAGTGGTTCCTC
CAGAAACCAGGTCAGTCCCCTAAGCTGCTCATCTACAAGGTCAGCAACCGGTTTAGCGGTGTA
CCAGACAGATTCTCTGGGTCCGGCTCCGGAACTGACTTTACCCTTAAGATCTCTAGGGTGGAA
GCCGAAGATCTCGGAGTGTACTACTGTTTCCAGGGGTCACATGTGCCACCTACTTTTGGAGGC
GGGACTAAGTTGGAGATAAAG
79: cAb-35 VH 
GAAGTGCAACTGCAGCAATCTGTCGCTGAACTCGTGAGACCTGGTGCATCTGTCAAATTGTCA
TGTACGGCATCAGGCTTTAACATTAAAAATACATACATGGATTGGGTCAAACAGCGGCCAGAA
CAGGGTCTGGAATGGATCGGAAGGATTGATCCAGCTAATGGTAACACCAAGCATGCTCCCAAG
TTTCAGGGAAAGGCCACCATTACCACTGATACAAGCAGCAATACTGCTTACCTGCAGCTGAGC
TCCTTGACCAGCGAGGACACCGCAATTTATTATTGTGCCAGGTGGGGAGGATGGGATGTGCGC
TTTGCCTACTGGGGCCAGGGTACCCTGGTAACTGTCTCCGCC
80: cAb-35 VL 
GACATCAAAATGACTCAGAGTCCTAGCAGTATGTACGCATCTCTCGGGGAGCGAGTTACCATC
AACTGCCAGGCCAGCCAAGATATCAACTCTTACCTGTCATGGTTCCAGCAGAAGCCCGGAAAG
AGTCCGAAGACCTTGATTCACCGAGCTAATAGGCTGGTGGATGGCGTGCCAAGCCGCTTCAGT
GGTAGTGGCTCTGGACAGGACTATTCACTGACCATTAGCAGCTTGGAATACGAGGATATGGGC
ATCTACTACTGTCTGCAATTTGACGAGTTCCCACTGACATTCGGCGCCGGCACAAAACTTGAG
TTGAAA
81: cAb-43 VH 
GAAGTCCAGCTTCAGCAGTCTGTTGCAGAACTGGTAAGACCTGGGGCCTCAGTAAAGTTGAG
CTGCACTGCTAGCGGCTTCAACATCCAGAACACCTACATCCACTGGGTGAAACAGAGACCCG
AACAAGGATTGGAGTGGATTGGCCGAATAGATCCCGCCTCCGGGAGTACTGAGTACGCTCCTA
AGTTCCAGGGCAAGGCCACAATCACAGCCGATACCTCCTCTAACAGCGGTTACCTGCAGCTGA
GCAGCTTGACCTCCGAGGATACTGCAATTTACTATTGTGCAAGATACGGCAACTTTCTGTATTAT
TACAGTATGGACTACTGGGGACAGGGAACCTCCGTCACTGTGTCTGCC
82: cAb-43 VL 
GACATCGTTATGACACAGGCTGCCCCTTCTGTTCCCGTAACACCAGGGGAGAGCGTGAGCATT
AGCTGTCGGTCAAACAAGTCTCTTCTGCACAGCAACGGAAATACCTATCTGTACTGGTTTCTG
CAGCGCCCAGGACAGAGTCCCCAGCTTTTGATCTACAGAATGAGTAACTTGGCCAGCGGGGT
GCCAGATCGCTTTTCCGGATCAGGAAGCGGCACCGCCTTTACCCTGCGGATTTCTCGGGTCGA
GGCAGAGGATGTGGGGGTGTACTACTGCATGCAGCACCTTGAAAATCCATACACTTTCGGGGG
AGGAACCAAGCTCGAGATCAAG
83: cAb-52 VH 
GAAGTACAGCTTCAACAGAGTGTTGCAGAGTTGGTGCGCCCTGGAGCCTCTGTGAAACTGAG
CTGCACAGCATCCGGTTTCAACATCCAGAATACTTACCTGCATTGGGTGAAGCAGCGGCCTGA
 
GCAGGGTCTGGAGTGGATTGGAAGAATCGACCCTGCAAATGATAATATCAAGTATGTGCCAAA
GTTCCAGGGCAAGGCAACCATTACAGCCGATACTAGCAGCAATACCGCCTACCTGCAGCTTTC
AAGCCTTACATCTGAGGACACTGCTATCTACTACTGCACAAGACACGGTCTGTTCTACGAGGG
GTGGTTTGCTTACTGGGGGCAGGGGACCCTTGTTACCGTGTCTACA
84: cAb-52 VL 
GAAATTGTACTGACACAGTCTCCAGCCTTGATGGCTGCCTCCCCTGGTGAGAAGGTGACTATT
ACCTGTAGCGTGAGCAGTTCTATTTCCAGCTCCAACCTGCACTGGTATCAGCAGAAGAGCGGC
ACCAGTCCAAAGCCCTGGATATATGGCACCTCCAACCTTGCATCCGGCGTGCCCGTGCGATTC
AGTGGCTCAGGTTCCGGTACTTCCTACTCTCTCACCATCTCCTCTATGGAAGCCGAAGACGCA
GCTACATACTACTGCCAGCAGTGGTCCAGTTACCCCTACACTTTCGGCTCTGGAACCAAGCTG
GAAATCAAG
85: hu-43VH-13: 
QVQLVQSGAEVKKPGASVKVSCKASGFNIQNTYIHWVRQAPGQGLEWIGRIDPASGSTEYAPKFQ
GRATITADTSTNTGYMELSSLRSEDTAVYYCARYGNFLYYYSMDYWGQGTLVTVSS
86: hu-43VH-21: 
QVQLVQSGAEVKKPGASVKVSCKASGFNIQNTYIHWVRQAPGQGLEWMGRIDPASGSTEYAPKF
QGRVTMTADTSINTGYMELSRLRSDDTAVYYCARYGNFLYYYSMDYWGQGTLVTVSS
87: hu-43VL-11: 
DIVMTQSPLSLPVTPGEPASISCRSNKSLLHSNGNTYLYWFLQKPGQSPQLLIYRMSNLASGVPDRF
SGSGSGTDFTLKISRVEAEDVGVYYCMQHLENPYTFGQGTKLEIK
88: hu-43VL-31 
DIVMTQTPLSLSVTPGQPASISCRSNKSLLHSNGNTYLYWFLQKPGQPPQLLIYRMSNLASGVPDR
FSGSGSGTDFTLKISRVEAEDVGVYYCMQHLENPYTFGQGTKLEI
89: hu-52VH-13 
QVQLVQSGAEVKKPGASVKVSCKASGFNIQNTYLHWVRQAPGQGLEWMGRIDPANDNIKYVPK
FQGRVTMTADTSTNTAYMELSSLRSEDTAVYYCTRHGLFYEGWFAYWGQGTLVTVSS
90: hu-52VH-23 
QVQLVQSGAEVKKPGASVKVSCKASGFNIQNTYLHWVRQAPGQGLEWMGRIDPANDNIKYVPK
FQGRVTMTADTSINTAYMELSRLRSDDTAVYYCTRHGLFYEGWFAYWGQGTLVTVSS
91: hu-52VH-33 
QVQLVQSGAEVKKPGSSVKVSCKASGFNIQNTYLHWVRQAPGQGLEWMGRIDPANDNIKYVPK
FQGRVTITADTSTNTAYMELSSLRSEDTAVYYCTRHGLFYEGWFAYWGQGTLVTVSS
92: hu-52VL-11 
DIQLTQSPSSLSASVGDRVTITCSVSSSISSSNLHWYQQKPGKAPKPLIYGTSNLASGVPSRFSGSGS
GTDYTLTISSLQPEDAATYYCQQWSSYPYTFGQGTKLEIK
93: hu-52VL-21 
DIQLTQSPSTLSASVGDRVTITCSVSSSISSSNLHWYQQKPGKAPKPLIYGTSNLASGVPS
RFSGSGSGTEYTLTISSLQPDDAATYYCQQWSSYPYTFGQGTKLEIK
94: hu-52VL-31 
EIVLTQSPATLSLSPGERATLSCSVSSSISSSNLHWYQQKPGQAPRPLIYGTSNLASGIPA
RFSGSGSGTDYTLTISSLEPEDAAVYYCQQWSSYPYTFGQGTKLEIK
95: Heavy chain constant region of IG4: 
ASTKGPSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVESKYGPPCPPCPAPEFLGGPSVFLFPPKPKDTLMI
SRTPEVTCVVVDVSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNHYTQKSLSLSLGK
96: Light chain constant region (Kappa): 
[?]


97: hu43-VL(N26S-N33S) VL: 
DIVMTQSPLSLPVTLGQPASISCRSSKSLLHSSGNTYLYWFQQRPGQSPRLLIYRMSNLASGVPDRF
SGSGSGTDFTLKISRVEAEDVGVYYCMQHLENPYTFGQGTKLEIK
98: hu43-VL(N26S-N33S) LCDR1:
   RSSKSLLHSSGNTYLY
99: hu43-VL(N26S-N33Q) VL: 
DIVMTQSPLSLPVTLGQPASISCRSSKSLLHSQGNTYLYWFQQRPGQSPRLLIYRMSNLASGVPDR
FSGSGSGTDFTLKISRVEAEDVGVYYCMOHLENPYTFGOGTKLEIK
100: hu43-VL(N26S-N33Q) LCDR1:
   RS**S**KSLLHS**Q**GNTYLY
101: hu43-VL(N26S-N33T) VL: 
DIVMTQSPLSLPVTLGQPASISCRSSKSLLHSTGNTYLYWFQQRPGQSPRLLIYRMSNLASGVPDRF
SGSGSGTDFTLKISRVEAEDVGVYYCMQHLENPYTFGQGTKLEIK
102: hu43-VL(N26S-N33T) LCDR1:
   RSSKSLLHSTGNTYLY
103: hu43-VL(N26S-G34A) VL: 
DIVMTQSPLSLPVTLGQPASISCRSSKSLLHSNANTYLYWFQQRPGQSPRLLIYRMSNLASGVPDRF
SGSGSGTDFTLKISRVEAEDVGVYYCMQHLENPYTFGQGTKLEIK
104: hu43-VL(N26S-N33T) LCDR1:
   RSSKSLLHSNANTYLY
105: hu43-VL(N26T-N33S) VL: 
DIVMTQSPLSLPVTLGQPASISCRSTKSLLHSSGNTYLYWFQQRPGQSPRLLIYRMSNLASGVPDRF
SGSGSGTDFTLKISRVEAEDVGVYYCMQHLENPYTFGQGTKLEIK
106: hu43-VL(N26T-N33S) LCDR1:
   RS**T**KSLLHS**S**GNTYLY
107: hu43-VL(N26T-N33Q) VL: 
DIVMTQSPLSLPVTLGQPASISCRSTKSLLHSQGNTYLYWFQQRPGQSPRLLIYRMSNLASGVPDR
FSGSGSGTDFTLKISRVEAEDVGVYYCMQHLENPYTFGQGTKLEIK
108: hu43-VL(N26T-N33Q) LCDR1:
   RS**T**KSLLHS**Q**GNTYLY
109: hu43-VL(N26T-N33T) VL: 
DIVMTQSPLSLPVTLGQPASISCRSTKSLLHSTGNTYLYWFQQRPGQSPRLLIYRMSNLASGVPDR
FSGSGSGTDFTLKISRVEAEDVGVYYCMQHLENPYTFGQGTKLEIK
110: hu43-VL(N26T-N33T) LCDR1:
   RS**T**KSLLHS**T**GNTYLY
111: hu43-VL(N26T-G34A) VL: 
DIVMTQSPLSLPVTLGQPASISCRSTKSLLHSNANTYLYWFQQRPGQSPRLLIYRMSNLASGVPDR
FSGSGSGTDFTLKISRVEAEDVGVYYCMQHLENPYTFGQGTKLEIK
112: hu43-VL(N26T- G34A) LCDR1:
   RS**T**KSLLHSN**A**NTYLY
113: hu43-VL(N26A-G34A) VL: 
DIVMTQSPLSLPVTLGQPASISCRSAKSLLHSNANTYLYWFQQRPGQSPRLLIYRMSNLASGVPDR
FSGSGSGTDFTLKISRVEAEDVGVYYCMQHLENPYTFGQGTKLEIK
114: hu43-VL(N26A- G34A) LCDR1:
   RS**A**KSLLHSN**A**NTYLY
115: hu43-VL(N26D-G34A) VL: 
DIVMTQSPLSLPVTLGQPASISCRSDKSLLHSNANTYLYWFQQRPGQSPRLLIYRMSNLASGVPDR
FSGSGSGTDFTLKISRVEAEDVGVYYCMQHLENPYTFGQGTKLEIK
116: hu43-VL(N26D- G34A) LCDR1:
   RS**D**KSLLHSN**A**NTYLY
117: hu43-VL(N26E-G34A) VL: 
[?]
DIVMTQSPLSLPVTLGQPASISCRSEKSLLHSNANTYLYWFQQRPGQSPRLLIYRMSNLASGVPDR
FSGSGSGTDFTLKISRVEAEDVGVYYCMQHLENPYTFGQGTKLEIK
118: hu43-VL(N26E- G34A) LCDR1:
   RS**E**KSLLHSN**A**NTYLY
119: hu43-VL(N26F-G34A) VL: 
DIVMTQSPLSLPVTLGQPASISCRSFKSLLHSNANTYLYWFQQRPGQSPRLLIYRMSNLASGVPDR
FSGSGSGTDFTLKISRVEAEDVGVYYCMQHLENPYTFGQGTKLEIK
120: hu43-VL(N26F- G34A) LCDR1:
   RS**F**KSLLHSN**A**NTYLY
121: hu43-VL(N26G-G34A) VL: 
DIVMTQSPLSLPVTLGQPASISCRSGKSLLHSNANTYLYWFQQRPGQSPRLLIYRMSNLASGVPDR
FSGSGSGTDFTLKISRVEAEDVGVYYCMQHLENPYTFGQGTKLEIK
122: hu43-VL(N26G- G34A) LCDR1:
   RS**G**KSLLHSN**A**NTYLY
123: hu43-VL(N26H-G34A) VL: 
DIVMTQSPLSLPVTLGQPASISCRSHKSLLHSNANTYLYWFQQRPGQSPRLLIYRMSNLASGVPDR
FSGSGSGTDFTLKISRVEAEDVGVYYCMQHLENPYTFGQGTKLEIK
124: hu43-VL(N26H- G34A) LCDR1:
   RS**H**KSLLHSN**A**NTYLY
125: hu43-VL(N26I-G34A) VL: 
DIVMTQSPLSLPVTLGQPASISCRSIKSLLHSNANTYLYWFQQRPGQSPRLLIYRMSNLASGVPDRF
SGSGSGTDFTLKISRVEAEDVGVYYCMQHLENPYTFGQGTKLEIK
126: hu43-VL(N26I- G34A) LCDR1:
   RS**I**KSLLHSN**A**NTYLY
127: hu43-VL(N26K-G34A) VL: 
DIVMTQSPLSLPVTLGQPASISCRSKKSLLHSNANTYLYWFQQRPGQSPRLLIYRMSNLASGVPDR
FSGSGSGTDFTLKISRVEAEDVGVYYCMQHLENPYTFGQGTKLEIK
128: hu43-VL(N26K- G34A) LCDR1:
   RS**K**KSLLHSN**A**NTYLY
129: hu43-VL(N26L-G34A) VL: 
DIVMTQSPLSLPVTLGQPASISCRSLKSLLHSNANTYLYWFQQRPGQSPRLLIYRMSNLASGVPDR
FSGSGSGTDFTLKISRVEAEDVGVYYCMQHLENPYTFGQGTKLEIK
130: hu43-VL(N26L- G34A) LCDR1:
   RS**L**KSLLHSN**A**NTYLY
131: VL: hud3-VL(N26P-G34A) VL: 
DIVMTQSPLSLPVTLGQPASISCRSPKSLLHSNANTYLYWFQQRPGQSPRLLIYRMSNLASGVPDR
FSGSGSGTDFTLKISRVEAEDVGVYYCMQHLENPYTFGQGTKLEIK
132: hu43-VL(N26P- G34A) LCDR1:
   RS**P**KSLLHSN**A**NTYLY
133: hu43-VL(N260-G34A) VL: 
DIVMTQSPLSLPVTLGQPASISCRSQKSLLHSNANTYLYWFQQRPGQSPRLLIYRMSNLASGVPDR
FSGSGSGTDFTLKISRVEAEDVGVYYCMOHLENPYTFGOGTKLEIK
134: hu43-VL(N26Q- G34A) LCDR1:
   RS**Q**KSLLHSN**A**NTYLY
135: VL:
hu43-VUN26R-G34A) VL: 
DIVMTQSPLSLPVTLGQPASISCRSRKSLLHSNANTYLYWFQQRPGQSPRLLIYRMSNLASGVPDR
FSGSGSGTDFTLKISRVEAEDVGVYYCMQHLENPYTFGQGTKLEIK
136: hu43-VL(N26R- G34A) LCDR1:
   RS**R**KSLLHSN**A**NTYLY
137: hu43-VL(N26V-G34A) VL: 
[?]
DIVMTQSPLSLPVTLGQPASISCRSVKSLLHSNANTYLYWFQQRPGQSPRLLIYRMSNLASGVPDR
FSGSGSGTDFTLKISRVEAEDVGVYYCMQHLENPYTFGQGTKLEIK
138: hu43-VL(N26V- G34A) LCDR1:
   RS**V**KSLLHSN**A**NTYLY
139: hu43-VL(N26W-G34A) VL: 
DIVMTQSPLSLPVTLGQPASISCRSWKSLLHSNANTYLYWFQQRPGQSPRLLIYRMSNLASGVPDR
FSGSGSGTDFTLKISRVEAEDVGVYYCMQHLENPYTFGQGTKLEIK
140: hu43-VL(N26W- G34A) LCDR1:
   RS**W**KSLLHSN**A**NTYLY
141: hu43-VL(N26Y-G34A) VL: 
DIVMTQSPLSLPVTLGQPASISCRSYKSLLHSNANTYLYWFQQRPGQSPRLLIYRMSNLASGVPDR
FSGSGSGTDFTLKISRVEAEDVGVYYCMQHLENPYTFGQGTKLEIK
142: hu43-VL(N26Y- G34A) LCDR1:
   RS**Y**KSLLHSN**A**NTYLY
143: hu43-VL(N26S-G34E) VL: 
DIVMTQSPLSLPVTLGQPASISCRSSKSLLHSNENTYLYWFQQRPGQSPRLLIYRMSNLASGVPDRF
SGSGSGTDFTLKISRVEAEDVGVYYCMQHLENPYTFGQGTKLEIK
144: hu43-VL(N26S-G34E) LCDR1:
   RS**S**KSLLHSN**E**NTYLY
145: hu43-VTL(N26S-G34G) VL: 
[?] VL(N28S G34G) VL:
DIVMTQSPLSLPVTLGQPASISCRSSKSLLHSNGNTYLYWFQQRPGQSPRLLIYRMSNLASGVPDR
FSGSGSGTDFTLKISRVEAEDVGVYYCMQHLENPYTFGQGTKLEIK
146: hu43-VL(N26S-G34G) LCDR1:
   RS**S**KSLLHSN**G**NTYLY
147: hu43-VL(N26S-G34K) VL: 
DIVMTQSPLSLPVTLGQPASISCRSSKSLLHSNKNTYLYWFQQRPGQSPRLLIYRMSNLASGVPDR
FSGSGSGTDFTLKISRVEAEDVGVYYCMQHLENPYTFGQGTKLEIK
148: hu43-VL(N26S-G34K) LCDR1:
   RS**S**KSLLHSN**K**NTYLY
149: hu43-VL(N26S-G34L) VL: 
DIVMTQSPLSLPVTLGQPASISCRSSKSLLHSNLNTYLYWFQQRPGQSPRLLIYRMSNLASGVPDRF
SGSGSGTDFTLKISRVEAEDVGVYYCMQHLENPYTFGQGTKLEIK
150: hu43-VL(N26S-G34L) LCDR1:
   RS**S**KSLLHSN**L**NTYLY
151: hud₃-VL(N26S-G34N) VL: 
[?]
DIVMTQSPLSLPVTLGQPASISCRSSKSLLHSNNNTYLYWFQQRPGQSPRLLIYRMSNLASGVPDRF
SGSGSGTDFTLKISRVEAEDVGVYYCMQHLENPYTFGQGTKLEIK
152: hu43-VL(N26S-G34N) LCDR1:
   RS**S**KSLLHSN**N**NTYLY
153: hu43-VL(N26S-G34Q) VL: 
[?]
DIVMTQSPLSLPVTLGQPASISCRSSKSLLHSNQNTYLYWFQQRPGQSPRLLIYRMSNLASGVPDR
FSGSGSGTDFTLKISRVEAEDVGVYYCMQHLENPYTFGQGTKLEIK
154: hu43-VL(N26S-G34Q) LCDR1:
   RS**S**KSLLHSN**Q**NTYLY
155: hu43-VL(N26S-G34R) VL: 
DIVMTQSPLSLPVTLGQPASISCRSSKSLLHSNRNTYLYWFQQRPGQSPRLLIYRMSNLASGVPDRF
SGSGSGTDFTLKISRVEAEDVGVYYCMQHLENPYTFGQGTKLEIK
156: hu43-VL(N26S-G34R) LCDR1:
   RS**S**KSLLHSN**R**NTYLY
157: hu43-VL(N26S-G34S) VL: 
DIVMTQSPLSLPVTLGQPASISCRSSKSLLHSNSNTYLYWFQQRPGQSPRLLIYRMSNLASGVPDRF
SGSGSGTDFTLKISRVEAEDVGVYYCMQHLENPYTFGQGTKLEIK
158: hu43-VL(N26S-G34S) LCDR1:
   RS**S**KSLLHSN**S**NTYLY
159: hu43-VL(N26S-G34T) VL: 
DIVMTQSPLSLPVTLGQPASISCRSSKSLLHSNTNTYLYWFQQRPGQSPRLLIYRMSNLASGVPDRF
SGSGSGTDFTLKISRVEAEDVGVYYCMQHLENPYTFGQGTKLEIK
160: hu43-VL(N26S-G34T) LCDR1:
   RS**S**KSLLHSN**T**NTYLY
161: Constant region mutation sequence of IG4: 
ASTKGPSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVESKYGPPCPPCPAPEFEGGPSVFLFPPKPKDTLMI
SRTPEVTCVVVAVSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLVKGFYPSDIAVEWE
SNGQPENNYKTTPPVLDSDGSFFLYSKLTVDKSRWQEGNVFSCSVMHEALHNHYTQKSLSLSLGK
162: Heavy chain sequence of hu43-VL(N26S-G34A)-IgG4 mutant antibody: 
QVQLVQSGAEVKKPGASVKVSCKASGFNIQNTYIHWVRQAPGQGLEWMGRIDPASGSTEYAPKF
QGRVTMTADTSTNTGYMELSSLRSEDTAVYYCARYGNFLYYYSMDYWGQGTLVTVSSASTKGPS
VFPLAPCSRSTSESTAALGCLVKDYFPEPVTVSWNSGALTSGVHTFPAVLQSSGLYSLSSVVTVPSS
SLGTKTYTCNVDHKPSNTKVDKRVESKYGPPCPPCPAPEFEGGPSVFLFPPKPKDTLMISRTPEVTC
VVVAVSQEDPEVQFNWYVDGVEVHNAKTKPREEQFNSTYRVVSVLTVLHQDWLNGKEYKCKVS
 
NKGLPSSIEKTISKAKGQPREPQVYTLPPSQEEMTKNQVSLTCLVKGFYPSDIAVEWESNGQPENN
YKTTPPVLDSDGSFFLYSKLTVDKSRWQEGNVFSCSVMHEALHNHYTQKSLSLSLGK
163: Light chain sequence of hu43-VL(N26S-G34A)-IaG4 mutant antibody: 
DIVMTQSPLSLPVTLGQPASISCRSSKSLLHSNANTYLYWFQQRPGQSPRLLIYRMSNLASGVPDRF
SGSGSGTDFTLKISRVEAEDVGVYYCMQHLENPYTFGQGTKLEIKRTVAAPSVFIFPPSDEQLKSGT
ASVVCLLNNFYPREAKVQWKVDNALQSGNSQESVTEQDSKDSTYSLSSTLTLSKADYEKHKVYA
CEVTHQGLSSPVTKSFNRGEC
164: Heavy chain of hu-52VH-33/VL11-IgG4 mutant antibody: 
QVQLVQSGAEVKKPGSSVKVSCKASGFNIQNTYLHWVRQAPGQGLEWMGRIDPANDNIKYVPK
FQGRVTITADTSTNTAYMELSSLRSEDTAVYYCTRHGLFYEGWFAYWGQGTLVTVSSASTKGPSV
FPLAPCSRSTSESTAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFEGGPSVFLFPPKPKDTLMISRTPEVTC
VVVAVSQEDPEVQFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVEWESNGQPENN
YKTTPPVLDSDGSFFLYSKLTVDKSRWQEGNVFSCSVMHEALHNHYTQKSLSLSLGK
165: Light chain of hu-52VH-33/VL11-IgG4 mutant antibody: 
DIQLTQSPSSLSASVGDRVTITCSVSSSISSSNLHWYQQKPGKAPKPLIYGTSNLASGVPSRFSGSGS
GTDYTLTISSLQPEDAATYYCQQWSSYPYTFGQGTKLEIKRTVAAPSVFIFPPSDEQLKSGTASVVC
LLNNFYPREAKVQWKVDNALQSGNSQESVTEQDSKDSTYSLSSTLTLSKADYEKHKVYACEVTH
QGLSSPVTKSFNRGEC
166: Heavy chain of hu43(N26S-G34A) antibody: 
QVQLVQSGAEVKKPGASVKVSCKASGFNIQNTYIHWVRQAPGQGLEWMGRIDPASGSTEYAPKF
QGRVTMTADTSTNTGYMELSSLRSEDTAVYYCARYGNFLYYYSMDYWGQGTLVTVSSASTKGPS
VFPLAPCSRSTSESTAALGCLVKDYFPEPVTVSWNSGALTSGVHTFPAVLQSSGLYSLSSVVTVPSS
SLGTKTYTCNVDHKPSNTKVDKRVESKYGPPCPPCPAPEFLGGPSVFLFPPKPKDTLMISRTPEVTC
VVVDVSQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLGK
167: Heavy chain of hu-52VH33/VL11 antibody:

QVQLVQSGAEVKKPGSSVKVSCKASGFNIQNTYLHWVRQAPGQGLEWMGRIDPANDNIKYVPK
FQGRVTITADTSTNTAYMELSSLRSEDTAVYYCTRHGLFYEGWFAYWGQGTLVTVSSASTKGPSV
FPLAPCSRSTSESTAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFLGGPSVFLFPPKPKDTLMISRTPEVTC
VVVDVSQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLGK

## Claims

1. An anti-NGK2A antibody or an antigen-binding fragment thereof, comprising a heavy chain variable region and a light chain variable region, the heavy chain variable region comprises three CDRs: VH-CDR1, VH-CDR2 and VH-CDR3, and the light chain variable region comprises three CDRs: VL-CDR1, VL-CDR2 and VL-CDR3; wherein,
the VH-CDR1 has an amino acid sequence as set forth in SEQ ID NO: 2;
the VH-CDR2 has an amino acid sequence as set forth in SEQ ID NO: 3;
the VH-CDR3 has an amino acid sequence as set forth in SEQ ID NO: 4;
the VL-CDR1 has an amino acid sequence as set forth in SEQ ID NO: 6;
the VL-CDR2 has an amino acid sequence as set forth in SEQ ID NO: 7;
the VL-CDR3 has an amino acid sequence as set forth in SEQ ID NO: 8; or
the VH-CDR1 has an amino acid sequence as set forth in SEQ ID NO: 10;
the VH-CDR2 has an amino acid sequence as set forth in SEQ ID NO: 11;
the VH-CDR3 has an amino acid sequence as set forth in SEQ ID NO: 12;
the VL-CDR1 has an amino acid sequence as set forth in SEQ ID NO: 14;
the VL-CDR2 has an amino acid sequence as set forth in SEQ ID NO: 15;
the VL-CDR3 has an amino acid sequence as set forth in SEQ ID NO: 16; or
the VH-CDR1 has an amino acid sequence as set forth in SEQ ID NO: 18;
the VH-CDR2 has an amino acid sequence as set forth in SEQ ID NO: 19;
the VH-CDR3 has an amino acid sequence as set forth in SEQ ID NO: 20;
the VL-CDR1 has an amino acid sequence as set forth in SEQ ID NO: 22;
the VL-CDR2 has an amino acid sequence as set forth in SEQ ID NO: 23;
the VL-CDR3 has an amino acid sequence as set forth in SEQ ID NO: 24; or
the VH-CDR1 has an amino acid sequence as set forth in SEQ ID NO: 26;
the VH-CDR2 has an amino acid sequence as set forth in SEQ ID NO: 27;
the VH-CDR3 has an amino acid sequence as set forth in SEQ ID NO: 28;
the VL-CDR1 has an amino acid sequence as set forth in SEQ ID NO: 30;
the VL-CDR2 has an amino acid sequence as set forth in SEQ ID NO: 31;
the VL-CDR3 has an amino acid sequence as set forth in SEQ ID NO: 32; or
the VH-CDR1 has an amino acid sequence as set forth in SEQ ID NO: 34;
the VH-CDR2 has an amino acid sequence as set forth in SEQ ID NO: 35;
the VH-CDR3 has an amino acid sequence as set forth in SEQ ID NO: 36;
the VL-CDR1 has an amino acid sequence as set forth in SEQ ID NO: 38;
the VL-CDR2 has an amino acid sequence as set forth in SEQ ID NO: 39;
the VL-CDR3 has an amino acid sequence as set forth in SEQ ID NO: 40; or
the VH-CDR1 has an amino acid sequence as set forth in SEQ ID NO: 42;
the VH-CDR2 has an amino acid sequence as set forth in SEQ ID NO: 43;
the VH-CDR3 has an amino acid sequence as set forth in SEQ ID NO: 44;
the VL-CDR1 has an amino acid sequence as set forth in SEQ ID NO: 46;
the VL-CDR2 has an amino acid sequence as set forth in SEQ ID NO: 47;
the VL-CDR3 has an amino acid sequence as set forth in SEQ ID NO: 48; or
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 54;
the VL-CDR2 has an amino acid sequence as set forth in SEQ ID NO: 55;
the VL-CDR3 has an amino acid sequence as set forth in SEQ ID NO: 56; or
the VH-CDR1 has an amino acid sequence as set forth in SEQ ID NO: 58;
the VH-CDR2 has an amino acid sequence as set forth in SEQ ID NO: 59;
the VH-CDR3 has an amino acid sequence as set forth in SEQ ID NO: 60;
the VL-CDR1 has an amino acid sequence as set forth in SEQ ID NO: 62;
the VL-CDR2 has an amino acid sequence as set forth in SEQ ID NO: 63;
the VL-CDR3 has an amino acid sequence as set forth in SEQ ID NO: 64; or
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 68;
the VL-CDR2 has an amino acid sequence as set forth in SEQ ID NO: 55;
the VL-CDR3 has an amino acid sequence as shown in SEQ ID NO: 56; and
when the VL-CDR1 has an amino acid sequence as set forth in SEQ ID NO: 68, X₁ is T, S, Q, A, D, G, E, F, I, K, L, P, V, W, Y, R or H; X₂ is N, Q, S or T; X₃ is G, A, L, E, K, R, S, N, T or Q; wherein, one and only one of the two conditions must exist: X₂ is N or X₃ is G; preferably, wherein:
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 100, 102, 104, 106, 108, 110, 112, 114, 116, 118, 120, 122, 124, 126, 128, 130, 132, 134, 136, 138, 140, 142, 144, 146, 148, 150, 152, 154, 156, 158 or 160;
the VL-CDR2 has an amino acid sequence as set forth in SEQ ID NO: 55;
the VL-CDR3 has an amino acid sequence as set forth in SEQ ID NO: 56; more preferably,
the VH-CDR1 has an amino acid sequence as set forth in SEQ ID NO: 50;
the VH-CDR2 has an amino acid sequence as set forth in SEQ ID NO: 51;
the VH-CDR3 has an amino acid sequence as set forth in SEQ ID NO: 52;
the VL-CDR1 has an amino acid sequence as set forth in SEQ ID NO: 104;
the VL-CDR2 has an amino acid sequence as set forth in SEQ ID NO: 55;
the VL-CDR3 has an amino acid sequence as set forth in SEQ ID NO: 56.

2. An anti-NGK2A antibody or an antigen-binding fragment thereof, comprising a heavy chain variable region and a light chain variable region, wherein:
the heavy chain variable region comprises three complementarity determining regions (CDRs) of a heavy chain variable region with an amino acid sequence as set forth in SEQ ID NO: 1, and the light chain variable region comprises three complementarity determining regions (CDRs) of a light chain variable region with an amino acid sequence as set forth in SEQ ID NO: 5; or
the heavy chain variable region comprises three complementarity determining regions (CDRs) of a heavy chain variable region with an amino acid sequence as set forth in SEQ ID NO: 9, and the light chain variable region comprises three complementarity determining regions (CDRs) of a light chain variable region with an amino acid sequence as set forth in SEQ ID NO: 13; or
the heavy chain variable region comprises three complementarity determining regions (CDRs) of a heavy chain variable region with an amino acid sequence as set forth in SEQ ID NO: 17, and the light chain variable region comprises three complementarity determining regions (CDRs) of a light chain variable region with an amino acid sequence as set forth in SEQ ID NO: 21; or
the heavy chain variable region comprises three complementarity determining regions (CDRs) of a heavy chain variable region with an amino acid sequence as set forth in SEQ ID NO: 25, and the light chain variable region comprises three complementarity determining regions (CDRs) of a light chain variable region with an amino acid sequence as set forth in SEQ ID NO: 29; or
the heavy chain variable region comprises three complementarity determining regions (CDRs) of a heavy chain variable region with an amino acid sequence as set forth in SEQ ID NO: 33, and the light chain variable region comprises three complementarity determining regions (CDRs) of a light chain variable region with an amino acid sequence as set forth in SEQ ID NO: 37; or
the heavy chain variable region comprises three complementarity determining regions (CDRs) of a heavy chain variable region with an amino acid sequence as set forth in SEQ ID NO: 41, and the light chain variable region comprises three complementarity determining regions (CDRs) of a light chain variable region with an amino acid sequence as set forth in SEQ ID NO: 45; or
the heavy chain variable region comprises three complementarity determining regions (CDRs) of a heavy chain variable region with an amino acid sequence as set forth in SEQ ID NO: 49, and the light chain variable region comprises three complementarity determining regions (CDRs) of a light chain variable region with an amino acid sequence as set forth in SEQ ID NO: 53; or
the heavy chain variable region comprises three complementarity determining regions (CDRs) of a heavy chain variable region with an amino acid sequence as set forth in SEQ ID NO: 57, and the light chain variable region comprises three complementarity determining regions (CDRs) of a light chain variable region with an amino acid sequence as set forth in SEQ ID NO: 61; or
the heavy chain variable region comprises three complementarity determining regions (CDRs) of a heavy chain variable region with an amino acid sequence as set forth in SEQ ID NO: 65, 85 or 86, and the light chain variable region comprises three complementarity determining regions (CDRs) of a light chain variable region with an amino acid sequence as set forth in SEQ ID NO: 66, 87, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159; or
the heavy chain variable region comprises three complementarity determining regions (CDRs) of a heavy chain variable region with an amino acid sequence as set forth in SEQ ID NO: 89, 90, or 91, and the light chain variable region comprises three complementarity determining regions (CDRs) of a light chain variable region with an amino acid sequence as set forth in SEQ ID NO: 92, 93, or 94; preferably,
the heavy chain variable region comprises three complementarity determining regions (CDRs) of a heavy chain variable region with an amino acid sequence as set forth in SEQ ID NO: 65, and the light chain variable region comprises three complementarity determining regions (CDRs) of a light chain variable region with an amino acid sequence as set forth in SEQ ID NO: 103; or
the heavy chain variable region comprises three complementarity determining regions (CDRs) of a heavy chain variable region with an amino acid sequence as set forth in SEQ ID NO: 91, and the light chain variable region comprises three complementarity determining regions (CDRs) of a light chain variable region with an amino acid sequence as set forth in SEQ ID NO: 92.

3. The anti-NGK2A antibody or the antigen-binding fragment thereof according to claim 1 or 2, wherein the heavy chain variable region has a sequence as set forth in SEQ ID NO: 1, or a sequence having at least 80%, 85%, 90%, 91%, 92%, 93%, 94%, 95%, 96%, 97%, 98% or 99% sequence identity to the sequence as set forth in SEQ ID NO: 1, and the light chain variable region has a sequence as set forth in SEQ ID NO: 5, or a sequence having at least 80%, 85%, 90%, 91%, 92%, 93%, 94%, 95%, 96%, 97%, 98% or 99% sequence identity to the sequence as set forth in SEQ ID NO: 5; or
the heavy chain variable region of the antibody or the antigen-binding fragment thereof has a sequence as set forth in SEQ ID NO: 9, or a sequence having at least 80%, 85%, 90%, 91%, 92%, 93%, 94%, 95%, 96%, 97%, 98% or 99% sequence identity to the sequence as set forth in SEQ ID NO: 9, and the light chain variable region has a sequence as set forth in SEQ ID NO: 13, or a sequence having at least 80%, 85%, 90%, 91%, 92%, 93%, 94%, 95%, 96%, 97%, 98% or 99% sequence identity to the sequence as set forth in SEQ ID NO: 13; or,
the heavy chain variable region of the antibody or the antigen-binding fragment thereof has a sequence as set forth in SEQ ID NO: 17, or a sequence having at least 80%, 85%, 90%, 91%, 92%, 93%, 94%, 95%, 96%, 97%, 98% or 99% sequence identity to the sequence as set forth in SEQ ID NO: 17, and the light chain variable region has a sequence as set forth in SEQ ID NO: 21, or a sequence having at least 80%, 85%, 90%, 91%, 92%, 93%, 94%, 95%, 96%, 97%, 98% or 99% sequence identity to the sequence as set forth in SEQ ID NO: 21; or,
the heavy chain variable region of the antibody or the antigen-binding fragment thereof has a sequence as set forth in SEQ ID NO: 25, or a sequence having at least 80%, 85%, 90%, 91%, 92%, 93%, 94%, 95%, 96%, 97%, 98% or 99% sequence identity to the sequence as set forth in SEQ ID NO: 25, and the light chain variable region has a sequence as set forth in SEQ ID NO: 29, or a sequence having at least 80%, 85%, 90%, 91%, 92%, 93%, 94%, 95%, 96%, 97%, 98% or 99% sequence identity to the sequence as set forth in SEQ ID NO: 29; or,
the heavy chain variable region of the antibody or the antigen-binding fragment thereof has a sequence as set forth in SEQ ID NO: 33, or a sequence having at least 80%, 85%, 90%, 91%, 92%, 93%, 94%, 95%, 96%, 97%, 98% or 99% sequence identity to the sequence as set forth in SEQ ID NO: 33, and the light chain variable region has a sequence as set forth in SEQ ID NO: 37, or a sequence having at least 80%, 85%, 90%, 91%, 92%, 93%, 94%, 95%, 96%, 97%, 98% or 99% sequence identity to the sequence as set forth in SEQ ID NO: 37; or,
the heavy chain variable region of the antibody or the antigen-binding fragment thereof has a sequence as set forth in SEQ ID NO: 41, or a sequence having at least 80%, 85%, 90%, 91%, 92%, 93%, 94%, 95%, 96%, 97%, 98% or 99% sequence identity to the sequence as set forth in SEQ ID NO: 41, and the light chain variable region has a sequence as set forth in SEQ ID NO: 45, or a sequence having at least 80%, 85%, 90%, 91%, 92%, 93%, 94%, 95%, 96%, 97%, 98% or 99% sequence identity to the sequence as set forth in SEQ ID NO: 45; or,
the heavy chain variable region of the antibody or the antigen-binding fragment thereof has a sequence as set forth in SEQ ID NO: 49, or a sequence having at least 80%, 85%, 90%, 91%, 92%, 93%, 94%, 95%, 96%, 97%, 98% or 99% sequence identity to the sequence as set forth in SEQ ID NO: 49, and the light chain variable region has a sequence as set forth in SEQ ID NO: 53, or a sequence having at least 80%, 85%, 90%, 91%, 92%, 93%, 94%, 95%, 96%, 97%, 98% or 99% sequence identity to the sequence as set forth in SEQ ID NO: 53; or,
the heavy chain variable region of the antibody or the antigen-binding fragment thereof has a sequence as set forth in SEQ ID NO: 57, or a sequence having at least 80%, 85%, 90%, 91%, 92%, 93%, 94%, 95%, 96%, 97%, 98% or 99% sequence identity to the sequence as set forth in SEQ ID NO: 57, and the light chain variable region has a sequence as set forth in SEQ ID NO: 61, or a sequence having at least 80%, 85%, 90%, 91%, 92%, 93%, 94%, 95%, 96%, 97%, 98% or 99% sequence identity to the sequence as set forth in SEQ ID NO: 61; or,
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 66, or a sequence having at least 80%, 85%, 90%, 91%, 92%, 93%, 94%, 95%, 96%, 97%, 98% or 99% sequence identity to the sequence as set forth in SEQ ID NO: 66; or,
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 67, or a sequence having at least 80%, 85%, 90%, 91%, 92%, 93%, 94%, 95%, 96%, 97%, 98% or 99% sequence identity to the sequence as set forth in SEQ ID NO: 67;
when the light chain variable region has an amino acid sequence as set forth in SEQ ID NO: 67, X₁ is T, S, Q, A, D, G, E, F, I, K, L, P, V, W, Y, R or H; X₂ is N, Q, S or T; X₃ is G, A, L, E, K, R, S, N, T or Q; wherein, one and only one of the two conditions must exist: X₂ is N or X₃ is G; or
the heavy chain variable region of the antibody or the antigen-binding fragment thereof has a sequence as set forth in SEQ ID NO: 85, or a sequence having at least 80%, 85%, 90%, 91%, 92%, 93%, 94%, 95%, 96%, 97%, 98% or 99% sequence identity to the sequence as set forth in SEQ ID NO: 85, and the light chain variable region has a sequence as set forth in SEQ ID NO: 87, or a sequence having at least 80%, 85%, 90%, 91%, 92%, 93%, 94%, 95%, 96%, 97%, 98% or 99% sequence identity to the sequence as set forth in SEQ ID NO: 87; or
the heavy chain variable region of the antibody or the antigen-binding fragment thereof has a sequence as set forth in SEQ ID NO: 86, or a sequence having at least 80%, 85%, 90%, 91%, 92%, 93%, 94%, 95%, 96%, 97%, 98% or 99% sequence identity to the sequence as set forth in SEQ ID NO: 86, and the light chain variable region has a sequence as set forth in SEQ ID NO: 87, or a sequence having at least 80%, 85%, 90%, 91%, 92%, 93%, 94%, 95%, 96%, 97%, 98% or 99% sequence identity to the sequence as set forth in SEQ ID NO: 87; or
the heavy chain variable region of the antibody or the antigen-binding fragment thereof has a sequence as set forth in SEQ ID NO: 65, or a sequence having at least 80%, 85%, 90%, 91%, 92%, 93%, 94%, 95%, 96%, 97%, 98% or 99% sequence identity to the sequence as set forth in SEQ ID NO: 65, and the light chain variable region has a sequence as set forth in SEQ ID NO: 88, or a sequence having at least 80%, 85%, 90%, 91%, 92%, 93%, 94%, 95%, 96%, 97%, 98% or 99% sequence identity to the sequence as set forth in SEQ ID NO: 88; or
the heavy chain variable region of the antibody or the antigen-binding fragment thereof has a sequence as set forth in SEQ ID NO: 89, 90, or 91, or a sequence having at least 80%, 85%, 90%, 91%, 92%, 93%, 94%, 95%, 96%, 97%, 98% or 99% sequence identity to the sequence as set forth in SEQ ID NO: 89, 90, or 91, and the light chain variable region has a sequence as set forth in SEQ ID NO: 92, 93 or 94, or a sequence having at least 80%, 85%, 90%, 91%, 92%, 93%, 94%, 95%, 96%, 97%, 98% or 99% sequence identity to the sequence as set forth in SEQ ID NO: 92, 93 or 94; preferably,
the heavy chain variable region of the antibody or the antigen-binding fragment thereof has a sequence as set forth in SEQ ID NO: 65, and the light chain variable region has a sequence as set forth in SEQ ID NO: 66, 87, 88, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157 or 159; or
the heavy chain variable region of the antibody or the antigen-binding fragment thereof has a sequence as set forth in SEQ ID NO: 91, and the light chain variable region has a sequence as set forth in SEQ ID NO: 92.

4. The anti-NGK2A antibody or the antigen-binding fragment thereof according to any one of claims 1 to 3, comprising a heavy chain constant region and/or a light chain constant region, wherein the heavy chain constant region and/or the light chain constant region are human-derived or murine-derived; preferably, the heavy chain constant region is a heavy chain constant region of human IgG4; more preferably, the heavy chain constant region comprises S228P, L235E, D265A and/or R409K mutations.

5. The anti-NGK2A antibody or the antigen-binding fragment thereof according to any one of claims 1 to 4, wherein the antibody comprises a heavy chain and a light chain, wherein,
the heavy chain has an amino acid sequence as set forth in SEQ ID NO: 162 or 166, or comprises an amino acid sequence having at least 80%, 85%, 90%, 91%, 92%, 93%, 94%, 95%, 96%, 97%, 98% or 99% sequence identity to SEQ ID NO: 162 or 166, and the light chain has an amino acid sequence as set forth in SEQ ID NO: 163, or comprises an amino acid sequence having at least 80%, 85%, 90%, 91%, 92%, 93%, 94%, 95%, 96%, 97%, 98% or 99% sequence identity to SEQ ID NO: 163; or
the heavy chain has an amino acid sequence as set forth in SEQ ID NO: 164 or 167, or comprises an amino acid sequence having at least 80%, 85%, 90%, 91%, 92%, 93%, 94%, 95%, 96%, 97%, 98% or 99% sequence identity to SEQ ID NO: 164 or 167, and the light chain has an amino acid sequence as set forth in SEQ ID NO: 165, or comprises an amino acid sequence having at least 80%, 85%, 90%, 91%, 92%, 93%, 94%, 95%, 96%, 97%, 98% or 99% sequence identity to SEQ ID NO: 165.

6. The anti-NGK2A antibody or the antigen-binding fragment thereof according to any one of claims 1 to 5, wherein the antibody is an IgG antibody, a bispecific antibody or a multispecific antibody; and the antigen-binding fragment is selected from Fab, Fab', (Fab')₂, Fv, Fvs linked by disulfide bonds, scFv and diabody.

7. A biological material, being:
1) a nucleic acid molecule encoding the anti-NGK2A antibody or the antigen-binding fragment thereof according to any one of claims 1 to 6; preferably, the nucleic acid molecule encodes the heavy chain variable region and/or the light chain variable region of the antibody or the antigen-binding fragment thereof; further preferably, the nucleic acid molecule encodes the heavy chain variable region with nucleotide sequence as set forth in SEQ ID NO: 69, 71, 73, 75, 77, 79, 81 or 83; and/or, the nucleic acid molecule encodes the light chain variable region with nucleotide sequence as set forth in SEQ ID NO: 70, 72, 74, 76, 78, 80, 82 or 84;
2) a vector comprising the nucleic acid molecule of 1) above; preferably, the vector is a bacterial plasmid, a phage, a yeast plasmid, a plant cell virus, a mammalian cell virus, or a retrovirus;
3) a cell comprising the nucleic acid molecule of 1) above or the vector of 2) above; preferably, the cell expresses the anti-NGK2A antibody or the antigen-binding fragment thereof according to any one of claims 1 to 6.

8. A method for preparing the anti-NGK2A antibody or the antigen-binding fragment thereof according to any one of claims 1 to 6, being:
1) chemical synthesis, synthesizing according to the amino acid sequence of the antibody or the antigen-binding fragment thereof; or,
2) biological preparation, including culturing the cell according to claim 7 and harvesting the anti-NGK2A antibody or the antigen-binding fragment thereof.

9. A composition comprising the anti-NGK2A antibody or the antigen-binding fragment thereof according to any one of claims 1 to 6, or the biological material according to claim 7;
preferably, the composition is a pharmaceutical composition, further including a pharmaceutically acceptable carrier.

10. A kit comprising the anti-NGK2A antibody or the antigen-binding fragment thereof according to any one of claims 1 to 6, or the biological material according to claim 7, or the composition according to claim 9; the kit is used for diagnosing or detecting NKG2A protein or cells expressing NKG2A protein in an *ex vivo* sample.

11. Use of the anti-NGK2A antibody or the antigen-binding fragment thereof according to any one of claims 1 to 6, or the biological material according to claim 7, or the composition according to claim 9, wherein the use is:
1) preparing a medicament for preventing and/or treating a disease associated with abnormal expression or abnormal function of NKG2A; or
2) preparing a medicament for enhancing the killing activity of NK92 cells or primary NK cells; or
3) preparing a medicament for stimulating human immune response; preferably,
the disease associated with abnormal expression or abnormal function of NKG2A is selected from a group consisting of cancer, viral disease, autoimmune disease or inflammatory disease; more preferably, the cancer is selected from a group consisting of non-small cell lung cancer, pharyngeal tumor, melanoma, colon cancer, lung cancer, liver cancer, pancreatic cancer, ovarian cancer, endometrial cancer, fallopian tube cancer, bladder cancer, glioma, glioblastoma, thyroid cancer, esophageal cancer, prostate cancer, breast cancer, lymphocytic leukemia, small cell lung cancer, head and neck cancer, head and neck squamous cell carcinoma, kidney cancer, gastric cancer, peritoneal tumor, aleukemic leukemia, acute lymphocytic leukemia, chronic lymphocytic leukemia, B cell lymphoma, T cell lymphoma, Hodgkin lymphoma, non-Hodgkin lymphoma, hairy cell lymphoma, Burketts lymphoma, acute myelogenous leukemia, chronic myelogenous leukemia and promyelocytic leukemia.
